(19) Europäisches Patentamt
European Patent Office
Office européen des brevets

(11) **EP 1 181 524 B1**

(12) **EUROPÄISCHE PATENTSCHRIFT**

(45) Veröffentlichungstag und Bekanntmachung des Hinweises auf die Patenterteilung:
**31.10.2012 Patentblatt 2012/44**

(21) Anmeldenummer: **00912444.7**

(22) Anmeldetag: **02.02.2000**

(51) Int Cl.:
***G01N 15/04*** (2006.01)    ***G01N 15/05*** (2006.01)

(86) Internationale Anmeldenummer:
**PCT/EP2000/000831**

(87) Internationale Veröffentlichungsnummer:
**WO 2000/046585 (10.08.2000 Gazette 2000/32)**

(54) **Verfahren zur An- oder Abreicherung von Tumorzellen aus einer Körperflüssigkeit und dazu geeigneter Kit**

Method for enriching or depleting tumour cells obtained from a body fluid and kit suitable for this purpose

Procédé pour enrichir ou appauvrir des céllules tumorales provenant d'un liquide organique et kit prévu à cet effet

(84) Benannte Vertragsstaaten:
**AT BE CH CY DE DK ES FI FR GB GR IE IT LI LU MC NL PT SE**

(30) Priorität: **03.02.1999 DE 19904267**

(43) Veröffentlichungstag der Anmeldung:
**27.02.2002 Patentblatt 2002/09**

(73) Patentinhaber: **Dahm, Michael W., Dr. Dr.**
**81677 München (DE)**

(72) Erfinder:
• **Dr. Dr. Dahm, Michael W.**
**81677 München (DE)**
• **Dr. Phelps, Robert C.**
**85757 Karlsfeld (DE)**

• **Dr. Brockmeyer, Carsten**
**83607 Holzkirchen (DE)**

(74) Vertreter: **Keller, Günter et al**
**Lederer & Keller**
**Patentanwälte**
**Unsöldstrasse 2**
**80538 München (DE)**

(56) Entgegenhaltungen:
EP-A- 0 566 252    EP-A- 0 875 202
WO-A-96/07097    WO-A-97/21488
US-A- 3 741 400    US-A- 3 887 464
US-A- 3 945 928    US-A- 5 270 171
US-A- 5 577 513    US-A- 5 663 051
US-A- 5 807 744    US-A- 5 840 502

**Beschreibung**

Die Erfindung betrifft ein Verfahren zur An- oder Abreicherung von Tumorzellen aus einer Körperflüssigkeit sowie einen dazu geeigneten Kit und ein Zentrifugationsgefäß.

Nahezu alle soliden malignen Tumore haben das Potential zur Metastasenbildung. Der Prozeß der Metastasierung beinhaltet die Aussaat maligner Zellen als Mikrometastasen, zumeist auf dem Blut- bzw. Lymphweg in entfernte Organe und die Ausbildung autonomer Tochtergeschwülste. Das Ausmaß der Filiarisierung bestimmt die Prognose eines Tumorleidens.

Die Ansprüche von Tumorvorsorge- oder Nachsorgeprogrammen liegen in der Früherkennung von Primärtumoren bzw. Rezidiven oder von Metastasen noch bevor diese klinisch manifest werden. Dieses Ziel kann bislang mit den verfügbaren apparativen Techniken nicht zufriedenstellend erfüllt werden. Durch den Nachweis von zirkulierenden Tumorzellen z.B. im peripheren Blut könnte frühzeitig, d.h. noch vor dem Auftreten eines klinisch bemerkten Tumors, eine möglicherweise kurative Immunmodulations- oder Polychemotherapie eingeleitet werden. Die Quantifizierung der Tumorzellen im peripheren Blut vor und nach der Therapie stellt dabei eine wichtige Kontrolle dar.

Da Körperflüssigkeiten im allgemeinen eine Vielzahl unterschiedlichster Zellen enthalten, ist es wünschenswert vor einer Quantifizierung bestimmter Zelltypen wie Tumorzellen, diese anzureichern und gleichzeitig eine möglichst große Menge von unerwünschten Zellen abzureichern, um die Quantifizierung zu erleichtern.

Darüber hinaus besteht ein erfolgversprechender Ansatz zur Quantifizierung von Tumorzellen in der Bestimmung der Telomerase-Aktivität einer Körperflüssigkeit. Beispielsweise beschreiben Kim et al. in Science (1994) 266: 2011 einen Assay, mit dem Telomerase-Aktivitäten in Tumorgeweben bestimmt werden können.

Im peripheren Blut weisen jedoch neben Tumorzellen auch hämatopoetische Stammzellen und aktivierte Lymphozyten eine erhöhte Telomerase-Aktivität auf. Aufgrund dieser Tatsache muß vor dem Nachweis einer Telomerase-Aktivität als Marker für disseminierte zirkulierende Tumorzellen im Blut eine Trennung der Telomerase-aktiven hämatopoetischen Stammzellen und Lymphozyten von den Tumorzellen vorgenommen werden.

Neben der Quantifizierung von Tumorzellen in Körperflüssigkeiten kann aber beispielsweise auch deren histologische Untersuchung unter einem Mikroskop von Interesse sein. Darüber hinaus können unter sterilen Bedingungen isolierte Tumorzellen in Kultur genommen werden, um daraus entsprechende Zellinien zu etablieren. Zellinien die von disseminierten zirkulierenden Tumorzellen abstammen anstatt von soliden Tumoren bieten die Möglichkeit, Prozesse der Metastasierung differenzierter untersuchen zu können. Darüber hinaus könnten diese Zellinien beispielsweise zur Entwicklung von effektiveren Tumortherapeutika beitragen.

Zur Anreicherung von Tumorzellen können beispielsweise mit Hilfe von spezifischen Antikörpern gegen Epithelzell-spezifische Antigene, wie z.B. EPCAM (Epithelial Cell Adhesion Molecule), HEA (Human Epithelial Antigen) und Cytokeratin 7/8, epitheliale Tumorzellen markiert werden und an magnetische Partikel oder fluoreszierende Moleküle gekoppelt, dann zur Anreicherung mittels eines Zell-Separators, wie MACS (Magnetic Cell Sorting) oder FACS (Fluorescence Activated Cell Sorting), verwendet werden. Diese Verfahren haben jedoch den Nachteil, daß nur Tumorzellen epithelialen Ursprungs erfaßt werden, nicht jedoch beispielsweise Melanomzellen. Außerdem sind diese Verfahren aufwendig und teuer.

In den 60er- und 70er-Jahren, als Methoden wie beispielsweise FACS oder MACS noch nicht zur Verfügung standen, wurden Tumorzellen von hämatopoetischen Zellen aufgrund ihrer unterschiedlichen Dichte getrennt (J.A. Fleming et al., J. clin. Path. (1967), 20, 145). Entsprechend dieser Daten besitzen Tumorzellen eine spezifische Dichte von 1,040-1,080, während Erythrozyten und polymorphnukleäre Leukozyten eine höhere Dichte aufweisen. Lymphozyten weisen hingegen eine spezifische Dichte im Bereich von 1,060-1,075 auf und überschneiden sich somit mit der spezifischen Dichte von Tumorzellen. Eine vollständige Abtrennung der ebenfalls Telomerase-aktiven Lymphozyten von den Tumorzellen über deren Dichteunterschiede sollte somit nicht möglich sein. So zeigte auch die Verwendung einer Standardlösung zur Isolierung von Lymphozyten, wie z.B. Histoprep® (Sigma) mit einer Dichte von 1,077 g/ml, daß Lymphozyten von gesunden Blutspendern mit einer Dichte von bis zu 1,077 g/ml eine Telomerase-Aktivität aufweisen.

US 5,663,051 offenbart eine Vorrichtung zur Anreicherung von spezifischen Zellarten aus Gemischen von Zellen. Dazu wird eine Zentrifugation in einem bestimmten Zellseperationsmedium mit einer bestimmten Dichte, nämlich 1,0490-1,0580 (für Tumorzellen) durchgeführt, wobei das Zentrifugationsgefäß als eine "Zellfalle" ausgebildet ist. Eine poröse Barriere zur Abtrennung verschiedener Phasen wird nicht offenbart.

Ein Zentrifugationsgefäß ist in US 5,840,502 beschrieben. Hierbei handelt es sich um ein Röhrchen, welches durch eine Querschnittsverengung in zwei Kompartimente unterteilt ist.

Die US 3,741,400 offenbart ein anderes Zentrifugationsgefäß für Blutproben mit zwei getrennten Kompartimenten für das Serum und Blutzellen. Während der Zentrifugation wird die Trennvorrichtung zwischen den Kompartimenten deformiert, so daß sie für Zellen durchlässig wird.

Diese Zentrifugationsgefäße unterscheiden sich jedoch von den erfindungsgemäß eingesetzten Zentrifugationsgefäßen.

Trotz umfangreicher Forschungen ist es bislang nicht gelungen, ein einfaches, schnelles und kostengünstiges Standardverfahren zur Tumorzellenanreicherung aus Körperflüssigkeiten zu entwickeln, bei dem auch Telomerase-aktive nicht-Tumorzellen abgetrennt werden.

Eine Aufgabe der vorliegenden Erfindung besteht somit darin, ein Verfahren zur An- oder Abreicherung von Tumorzellen aus einer Körperflüssigkeit zur Verfügung zu stellen, das die obengenannten Nachteile nicht aufweist und insbesondere auch in der Lage ist, nicht-Tumorzellen, die eine Telomerase-Aktivität aufweisen, von den gewünschten Tumorzellen abzutrennen.

Es wurde nun überraschend gefunden, daß diese Aufgabe dadurch gelöst werden kann, daß man ein Zellseparationsmedium mit einer Dichte im Bereich von 1,059 bis 1,065 g/ml mit der Körperflüssigkeit, die Tumorzellen enthält, überschichtet und zentrifugiert. Durch die Verwendung des speziellen Zellseparationsmediums werden die in der Körperflüssigkeit vorhandenen Zellen so aufgetrennt, daß die aufgrund ihrer Dichte zusammen mit den Tumorzellen angereicherten Lymphozyten keine Telomerase-Aktivität aufweisen.

Die vorliegende Erfindung betrifft somit ein Verfahren nach Anspruch 1 zur An- oder Abreicherung von Tumorzellen aus einer Körperflüssigkeit, worin ein Zellseparationsmedium mit der Körperflüssigkeit überschichtet und zentrifugiert wird, dadurch gekennzeichnet, daß das Zellseparationsmedium eine Dichte im Bereich von 1,059 bis 1,065 g/ml aufweist.

Es versteht sich, daß das erfindungsgemäße Verfahren in Abhängigkeit davon, mit welcher Fraktion nach der Zentrifugation weitergearbeitet wird, sowohl zur An- als auch zur Abreicherung von Tumorzellen eingesetzt werden kann. Im folgenden wird daher nicht zwischen diesen beiden möglichen Weiterbehandlungen unterschieden sondern allgemein von Anreicherung gesprochen, wobei erfindungsgemäß jedoch stets beide Möglichkeiten umfaßt sind.

Das Anreicherungsverfahren verwendet ein Zellseparationsmedium als diskontinuierlichen Gradienten, das mit der Körperflüssigkeit überschichtet wird. Durch Zentrifugation werden die Zellen ihrer spezifischen Zelldichte nach aufgetrennt und können in einzelnen Fraktionen abgenommen werden. Der spezifische Dichtegrad des Zellseparationsmediums erlaubt eine fast vollkommene Trennung von Tumorzellen von den in den Körperflüssigkeiten befindlichen korpuskulären Anteilen, speziell den Zellen des roten und weißen Blutsystems. Darüber hinaus erlaubt es das Verfahren, Telomerase-positive von Telomerase-negativen hämatopoetischen Zellen zu trennen, wobei sich die angereicherten Tumorzellen nach der Zentrifugation in der gleichen Fraktion befinden wie die Telomerase-negativen hämatopoetischen Zellen, so daß eine anschließend nachgewiesene Telomerase-Expression in dieser Fraktion zweifelsfrei auf vorhandene Tumorzellen zurückgeführt werden kann.

Überraschend ist auch, daß durch die gegenüber dem Stand der Technik nur geringfügige Verringerung der Dichte des Zellseparationsmediums eine erhebliche Reduzierung der kontaminierenden Blutzellen erreicht wird. Dadurch wird die Gesamt-Zellzahl signifikant verringert, ohne daß signifikante Verluste an Tumorzellen auftreten, wodurch z.B. das Screening von mikroskopischen Präparaten erheblich vereinfacht und im klinischen Maßstab erst möglich wird.

Es wurde gefunden, daß eine besonders gute Trennleistung, im Sinne einer Anreicherung von Tumorzellen und einer gleichzeitigen Abreicherung von unerwünschten Blutzellen mit einem Zellseparationsmedium einer Dichte im Bereich von 1,059-1,065 g/ml, bevorzugt von 1,059-1,062 g/ml und besonders bevorzugt von etwa 1,060 g/ml, insbesondere 1,060 g/ml $\pm$ 0,0005 g/ml erzielt wird.

Es wurde auch gefunden, daß die Trennleistung des Zellseparationsmediums auch von dem Alter des Blutes nach Blutentnahme, und von der Konzentration der dem Blut beigesetzten Antikoagulanzien abhängig ist. Es wurde auch gefunden, daß bei Frischblut, d.h. Blut, das noch am gleichen Tag (= 24 Stunden) der Blutabnahme untersucht wird, eine besonders gute Trennleistung mit einem Zellseparationsmedium im besonders bevorzugten Bereich von etwa 1,060 g/ml $\pm$ 0,0005 g/ml erzielt wird.

Die Zentrifugation wird vorteilhaft bei ca. 500 bis 2.000 x g, bevorzugt bei ca. 1.000 x g über ca. 10 bis 30 Minuten, bevorzugt über 20-30 Minuten durchgeführt. Die Temperatur während der Zentrifugation beträgt vorzugsweise ca. 4°C. Dies bewirkt, daß die katalytische Aktivität von Proteasen, DNAsen bzw. RNAsen möglichst gering gehalten wird.

Als Zellseparationsmedium läßt sich im Prinzip jede geeignete Flüssigkeit gewünschter Dichte verwenden. Das Zellseparationsmedium sollte nicht mit der Körperflüssigkeit oder den darin enthaltenen Zellen reagieren. Vorteilhaft kann beispielsweise Ficoll oder Percoll bzw. ein percoll- oder ficollähnliches Medium verwendet werden, wobei die Lösungen jeweils nach Anweisungen des Herstellers auf die gewünschte Dichte gebracht werden. Beispielsweise berechnet sich die Menge der zu verdünnenden Percoll-Stammlösung mit einer Dichte von 1,13 g/ml, die zur Herstellung von 100 ml einer Percoll-Arbeitslösung gewünschter Dichte (dd) benötigt wird, nach der Formel:

$$100 \text{ ml} \times (dd - 0{,}106 - 0{,}9)/0{,}13.$$

10% der Percoll-Arbeitslösung gewünschter Dichte bestehen immer aus einer 10 x physiologischen Lösung, wie z.B. 10 x PBS (Phosphat gepufferte Salzlösung), oder aus einer 1,5 M NaCl-Lösung, um eine physiologische Osmolarität zu gewährleisten. Die Differenz zwischen der nach der vorstehenden Formel berechneten Menge an Percoll-Stammlösung (Dichte 1,13 g/ml) und der Salzlösung zu 100 ml wird dann mit Wasser aufgefüllt.

Damit läßt sich eine Percoll-Arbeitslösung mit einer Dichte von 1,060 g/ml beispielsweise wie folgt herstellen:

| | |
|---|---|
| 41,54 ml | der Percoll-Stammlösung (Dichte von 1,13 g/ml) |
| 48,46 ml | $H_2O$ |
| <u>10,00 ml</u> | <u>1,5 M NaCl</u> |

100,00 ml Percoll-Arbeitslösung, dd 1,060 g/ml

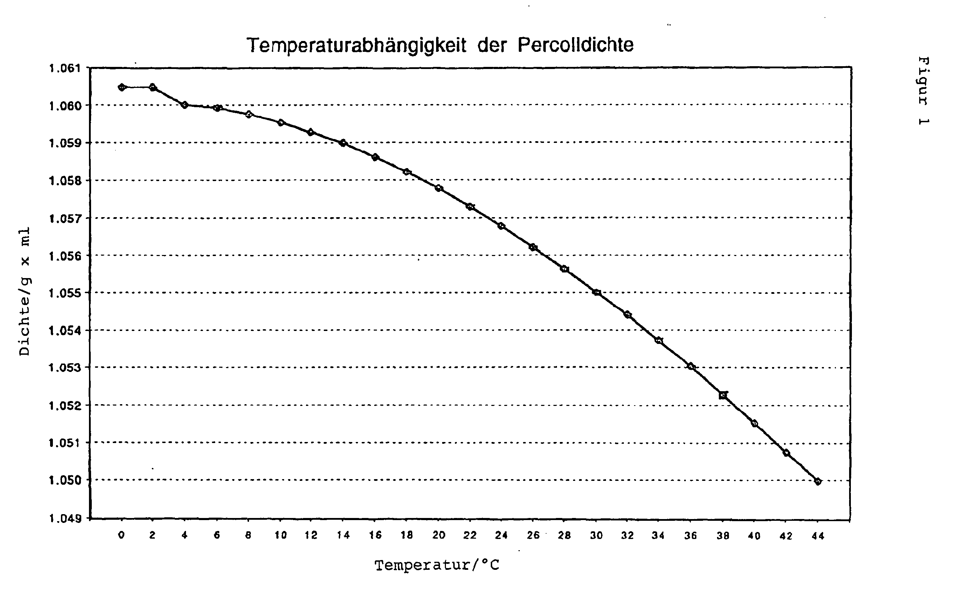

Vorteilhaft wird die Dichte des Zellseparationsmediums mit Hilfe eines Dichtemeßgerätes (DMA 4500, Anton Paar, Österreich) bei der entsprechenden Arbeitstemperatur von 44°C eingestellt. Bei der anschließenden Zellseparation sollte darauf geachtet werden, daß eine Umgebungs- bzw. Arbeitstemperatur von 8°C nicht überschritten wird. Dies würde zu einer signifikanten Erniedrigung der Percoll-Dichte (vgl. Figur 1) und zu unerwünschtem Zellverlust führen.

Bei der Körperflüssigkeit, aus der Tumorzellen angereichert werden sollen, kann es sich um jede menschliche oder tierische Körperflüssigkeit oder um eine Dispersion von Zellgewebe handeln. Hierbei handelt es sich beispielsweise um Blut, insbesondere peripheres Blut wie venöses oder arterielles Blut, Lymphe, Urin, Exsudate, Transudate, Spinalflüssigkeit, Samenflüssigkeit, Speichel, Flüssigkeiten aus natürlichen oder unnatürlichen Körperhöhlen, Knochenmark und dispergiertes Körpergewebe. Bei den Flüssigkeiten aus natürlichen Körperhöhlen kann es sich beispielsweise um seröse Flüssigkeiten wie Peritoneal- und Pleuraflüssigkeiten handeln, bei den Flüssigkeiten aus unnatürlichen Körperhöhlen kann es sich beispielsweise um Flüssigkeiten aus Zysten handeln.

Bevorzugte Körperflüssigkeiten sind Blut, Knochenmark, Lymphe, seröse Flüssigkeiten aus Körperhöhlen sowie Urin, wobei Blut und Urin besonders bevorzugt sind. Urin eignet sich insbesondere zur Anreicherung von Zellen von Blasentumoren.

Die bevorzugteste Körperflüssigkeit ist jedoch peripheres Blut, das vorteilhaft in einer gerinnungshemmenden Substanz abgenommen und vor dem Überschichten des Zellseparationsmediums mit einem Verdünnungsmittel verdünnt wird. Als gerinnungshemmende Substanzen können beispielsweise EDTA oder Citrat bzw. Heparin oder CPD (Citrat, Phosphat, Dextrose) oder vergleichbare Substanzen eingesetzt werden. Als peripheres Blut eignet sich venöses oder arterielles Blut.

Die zu untersuchende Körperflüssigkeit wird entsprechend gängiger Standardprotokolle entnommen bzw. gesammelt. In Abhängigkeit von der Art der Körperflüssigkeit wird diese dann entweder zunächst mit einem Verdünnungsmittel, bevorzugt einem Puffer, verdünnt oder direkt unverdünnt in einem Zentrifugationsgefäß über das Zellseparationsmedium geschichtet. Alternativ kann die Körperflüssigkeit zuvor bei beispielsweise 1.000 x g für ca. 10 Minuten zentrifugiert und nach Resuspension der Zellen in einem Puffer über das Zellseparationsmedium geschichtet werden. Der bevorzugt verwendete Puffer ist Dulbecco PBS. Als Zentrifugationsgefäß eignet sich insbesondere ein Zentrifugationsgefäß, das aus Kunststoff, wie z.B. Polypropylen hergestellt ist, um einer unspezifischen Adsorption von Zellen vorzubeugen. Das Zentrifugationsgefäß sollte verschließbar sein.

In einer bevorzugten Ausführungsform des Verfahren werden der Körperflüssigkeit vor dem Überschichten eine oder mehrere Substanzen zugegeben, die eine Aggregation von Thrombozyten an Tumorzellen verhindern. Diese Substanzen können zum Beispiel mit dem als Verdünnungsmittel verwendeten Puffer zugesetzt werden. Als Substanzen, die eine unerwünschte Aggregation von Thrombozyten an Tumorzellen verhindern, eignen sich beispielsweise EDTA, Citrat und ACD-A (acid citrate dextrose). Zusätzlich oder statt dessen kann die Körperflüssigkeit vor dem Überschichten von Substanzen befreit werden, die eine Aggregation von Thrombozyten an Tumorzellen fördern. Hierbei handelt es sich beispielsweise um Ionen wie Magnesium- und Calciumionen.

Im Zentrifugationsgefäß wird das Zellseparationsmedium, das eine höhere Dichte als die zu untersuchende Körperflüssigkeit aufweist, vorgelegt, und anschließend mit der Körperflüssigkeit überschichtet. Je nach Größe des Zentrifugationsgefäßes und dem Volumen der Körperflüssigkeit, aus der die Tumorzellen angereichert werden sollen, kann das Zellseparationsmedium beispielsweise mit einem Volumen von 1-500 ml vorgelegt werden.

Als besonders vorteilhaft hat es sich erwiesen, wenn das untere Viertel des Zentrifugationsgefäßes nach der Zentrifugation und vor der Abnahme der an Tumorzellen angereicherten Interphase kurzzeitig stark abgekühlt wird um Zell-Kontaminationen zu verhindern. Beispielsweise können die im Zellpellet befindlichen Erythrozyten und Leukozyten dadurch immobilisiert werden, daß das untere Viertel des Zentrifugationsgefäßes für 5-10 Minuten in flüssigem Stickstoff stark abgekühlt wird. Als Interphase wird vorliegend der Übergang zwischen dem Zellseparationsmedium und der darüberliegenden Körperflüssigkeit bezeichnet. In dieser Interphase reichern sich die Tumorzellen an und werden nach der Zentrifugation beispielsweise durch Absaugen dieser Phase gesammelt. Durch das starke Abkühlen des Zentrifugationsgefäßes wird ein Vermischen der Zellen aus den verschiedenen Phasen verhindert, wodurch falsch-positive Testergebnisse ausgeschlossen werden können.

Um einen möglichst einfachen Ablauf der Arbeiten zu sichern, kann in einer bevorzugten Ausführungsform des erfindungsgemäßen Verfahrens die Zentrifugation in einem Gefäß durchgeführt werden, das durch eine Barriere, einen Filter oder ein Sieb, nachfolgend poröse Barriere oder Barriere genannt, in ein oberes und ein unteres Kompartiment geteilt ist, wobei das Zellseparationsmedium im unteren Kompartiment vorgelegt und die Körperflüssigkeit in das obere Kompartiment eingebracht wird. Hierdurch wird ein Vermischen der zu untersuchenden Körperflüssigkeit im oberen Kompartiment mit dem Zellseparationsmedium im unteren Kompartiment vor und nach dem Zentrifugationsschritt vermieden.

Die Position der porösen Barriere in dem Zentrifugationsgefäß kann dabei so gewählt werden, daß der Flüssigkeitsspiegel des Zellseparationsmediums entweder genau unterhalb, genau innerhalb oder genau oberhalb der porösen Barriere zu liegen kommt.

Die poröse Barriere kann beispielsweise eine Dicke von 0,5-10 mm, vorzugsweise von 1-5 mm aufweisen. Die poröse Barriere sollte darüber hinaus eine Festigkeit aufweisen, die es ihr erlaubt den Zentrifugationskräften unbeschadet Stand zu halten. Eine bevorzugte Verwendung finden Barrieren mit einer porösen Beschaffenheit, die es erlaubt, daß bei der Zentrifugation Flüssigkeiten sowie die korpuskulären Bestandteile des Blutes, wie die Zellen des roten und weißen Blutsystems, welche eine höhere Dichte als das vorgelegte Zellseparationsmedium haben, die poröse Barriere ungehindert passieren können. Dies hat zur Folge, daß das Zellseparationsmedium während der Zentrifugation durch die poröse Membran in das obere Kompartiment gedrängt wird und die Tumorzellen und Thrombozyten welche eine geringere Dichte als das vorgelegte Zellseparationsmedium haben, auf einer Ebene oberhalb der Barriere zu liegen kommen. Eingesetzt werden poröse Barrieren mit einer Porengröße von 20-100 $\mu$m, vorzugsweise 20-30 $\mu$m.

Die poröse Barriere kann aus jedem geeigneten Material bestehen. Beispielsweise eignen sich Kunststoff, Metall, Keramik oder eine Mischung oder spezielle Legierung dieser Stoffe. Es kann aber auch jedes andere natürliche oder künstliche, geeignete Material eingesetzt werden.

In einer ebenfalls bevorzugten Ausführungsform des erfindungsgemäßen Verfahrens besteht die poröse Barriere aus einem hydrophoben Material oder ist mit einem hydrophoben Material beschichtet.

Mit Hilfe der porösen Barriere kann die zu untersuchende Körperflüssigkeit in das Zentrifugationsgefäß gefüllt werden, ohne daß sie sich mit dem darunterliegenden Zellseparationsmedium vermischt und somit die Anreicherung beeinträchtigen oder unmöglich machen kann.

Nach der Zentrifugation befinden sich die in der Körperflüssigkeit vorhandenen Tumorzellen in der Interphase des oberen Kompartiments des Zentrifugationsgefäßes. Die Flüssigkeit oberhalb der Interphase kann dann zu etwa 80% vorsichtig abgesaugt und verworfen werden. Bei dieser Restflüssigkeit handelt es sich beispielsweise bei der Verwendung von Blut als Körperflüssigkeit um Plasma, ein Plasma/PBS-, bzw. Plasma/Puffer-Gemisch, das die Proteine des Plasmas enthält.

Der verbleibende Überstand oberhalb der Barriere, in dem sich die Tumorzellen befinden, kann anschließend gesammelt und beispielsweise in ein frisches Zentrifugationsgefäß (vorzugsweise aus gegebenenfalls silikonisiertem Kunststoff und mit der gleichen Volumenkapazität wie das zuvor verwendete Zentrifugationsgefäß) überführt werden. Die poröse Barriere verhindert bei der Entnahme des verbleibenden Überstands ein Vermischen der Zellen des oberen und des unteren Kompartiments.

Vorteilhaft wird anschließend das obere Kompartiment des Zentrifugationsgefäßes beispielsweise zweimal mit einem Puffer nachgewaschen und dieser wird ebenfalls in das frische Zentrifugationsgefäß überführt. Als Puffer eignen sich beispielsweise Dulbeccos PBS (3,9 mM EDTA, pH 8,0 ohne Calcium und Magnesium) oder NaCl/10% ACD-A (Guidelines for the collection, processing and storage of human bone marrow and peripheral stem cells for transplantation, prepared by the BCSH Blood Transfusion Task. Transfus. Med. 1994; 4: 165-72) oder NaCl/5% ACD-A/1% Albumin). Als besonders vorteilhaft hat es sich erwiesen, dem Puffer Proteine, wie z.B. 0.1% bis 10% BSA (Bovines Serum Albumin) zuzusetzen, da unspezifische Bindungen der zu sammelnden Zellen an Oberflächen wie Gefäßwand, Barriere bzw. Klappe, Pipettenspitzen etc. verhindert werden. Nach einer weiteren Zentrifugation beispielsweise bei 1.000 x g über ca. 10 Minuten bei einer Temperatur von ca. 4°C können die gesammelten Zellen beispielsweise den Tumorzellnachweismethoden zugeführt werden.

Nachdem in der gesammelten Tumorzellfraktion auch Thrombozyten angereichert werden, kann es vorteilhaft sein, die Thrombozyten noch zweimal in einem Puffer (z.B. PBS mit 0,1% - 10% BSA) zu waschen und bei ca. 200 x g und 10 Minuten zu zentrifugieren, wenn die gesammelten Zellen z. B. auf Objektträger aufgebracht werden sollen.

Um den nach der Zentrifugation die Tumorzellen enthaltenden Zellring an der Interphase zwischen Zellseparationsmedium und Körperflüssigkeit für die Entnahme aus dem Zentrifugationsgefäß besser sichtbar zu machen, hat es sich als vorteilhaft erwiesen, dem Zellseparationsmedium einen Farbstoff zuzugeben. Beispielsweise können zu 100 ml einer Percoll-Arbeitslösung 100 $\mu$l einer 1% Trypan-Blau-Lösung zugegeben werden.

Bei Verwendung eines Zentrifugationsgefäßes mit poröser Barriere wird nach Entnahme der überstehenden Restflüssigkeit oberhalb der Interphase jedoch bevorzugt nicht nur die Interphase sondern die gesamte verbliebene Flüssigkeit oberhalb der porösen Barriere entnommen, nicht weil sich zwischen Interphase und poröser Barriere noch weitere Zellen befinden, sondern weil durch die Abnahme die im Zellring enthaltenen Zellen leicht durchmischt werden können. Um keine Zellen aus dem oberen Kompartiment zu verlieren wird dieses vorteilhaft noch zweimal mit einem Puffer (z.B. PBS mit 0,1% - 10% BSA) gewaschen.

Mit dem erfindungsgemäßen Verfahren können zirkulierende Tumorzellen und insbesondere zirkulierende Tumorzellen von soliden Tumoren, d.h. von nicht-hämatologischen Tumoren, angereichert werden oder hämatologische Tumorzellen, d.h. Tumorzellen des roten und weißen Blutsystems.

Mit dem erfindungsgemäßen Tumorzellanreicherungsverfahren können Tumorzellen aufgrund ihrer unterschiedlichen Dichte nahezu vollständig von den korpuskulären Bestandteilen von Körperflüssigkeiten wie z.B. den Zellen des roten und weißen Blutsystems getrennt, konzentriert und beispielsweise einer der bekannten Tumorzellennachweisverfahren zugeführt werden.

Die Methoden zum Nachweis von Tumorzellen umfassen das gesamte Spektrum der gängigen Diagnostik. Beispiele hierfür sind die mikroskopischen, immunzytologischen/immunzytochemischen, biochemischen und/oder molekularbiologischen Verfahren. Beispielsweise können die Tumorzellen nach der Anreicherung als ganze Zellen oder als Zellbestandteile direkt oder nach Zellkultur und Expansion der Tumorzellen durch morphologische, immunzytologische/immunzytochemische, biochemische und/oder molekularbiologische Verfahren nachgewiesen werden. Diese Verfahren ermöglichen den Nachweis einer ganzen Zelle, der spezifischen Aktivität einer Zelle oder den Nachweis von spezifischen Bestandteilen einer ganzen Zelle. Beispiele von Bestandteilen einer ganzen Zelle sind u.a. Proteine und Glykoproteine der Membran, des Zytoplasmas oder des Zellkerns, sowie die Chromosomen, spezifische Abschnitte von Chromosomen bis hin zu Nukleinsäuresequenzen, wie DNA, RNA und cDNA.

Beispiele direkter Zellnachweisverfahren sind u.a. sämtliche Formen der Mikroskopie einschließlich der Färbung von Zellen oder Zellbestandteilen. Beispiele direkter Färbung sind die Trypan-Blau-Färbung oder die Färbung durch spezifische Antikörper, die gegen spezifische Bestandteile der Zelle, wie der Zellmembran, das Zytoplasma oder der Zellkern gerichtet sind und an denen Markierungssignale wie z.B. fluoreszierende Farbstoffe gekoppelt sind. Nachweisverfahren sind u.a. Durchflußzytometrie oder FACS (Fluorescence activated cell sorting), ELISA und Western Blotting. Weitere Verfahren zum Nachweis von Zellbestandteilen sind u.a. die Detektionsverfahren von Nukleinsäuren mit Hilfe von markierten Sonden, z. B. FISH, in situ Hybridisierung, Northern, South-Western und Southern Blotting oder differential display sowie u.a. die Amplifikationsverfahren von Nukleinsäuren u.a. die PCR, RT-PCR, in situ RT-PCR und NASBA.

Weiterhin ist es vorteilhaft, Verfahren einzusetzen, die eine spezifische Bindung eines Antikörpers an ein Protein oder eines Oligonukleotids an eine Nukleinsäure (DNA, RNA oder cDNA) durch eine Signalamplifizierung sichtbar machen. Ein Beispiel hierfür ist der Einsatz spezifischer Dendrimere (Polyprobe) (vgl. US Patent 5,487,973 und Nilsen, T.W., Grayzel, J. und Prensky, W. (1997) J. theor. Biol. 187: 273-284). Dendrimere sind hochverzweigte Strukturen, die vorzugsweise aus Nukleinsäuren bestehen und aus der sequenziellen Hybridisierung monomerer Strukturen hervorgehen. Ein Monomer ist ein Heterodimer, der aus zwei Einzelstrang-Nukleinsäuren besteht, die eine Doppelstrang-Taile und vier Einzelstrang-Armen bilden. Ein Dendrimer ist aus der sequenziellen Bindung solcher Monomere in mehreren Bindungsebenen zusammengesetzt: Die erste Bindungsebene besteht aus vier Monomeren mit zwölf Einzelstrang-Armen. Die zweite Ebene besteht aus zwölf Monomeren mit 36 Einzelstrang-Armen. Die sechste Ebene besteht aus 972 Monomeren mit 2916 freien Einzelstrang-Armen. An einen der Einzelstrang-Arme können zum einen spezifische Antikörper oder spezifische Sonden wie Nukleinsäuren angekoppelt werden. An den anderen Einzelstrang-Arm hingegen können spezifische Markierungssignale wie z.B. fluoreszierende Farbstoffe, oder radioaktive Substanzen gekoppelt werden. Der Einsatz der Dendrimere zum Nachweis von seltener DNA oder RNA kann entweder direkt oder indirekt nach Amplifikation durch z.B. PCR, RT-PCR oder NASBA erfolgen.

Die angeschlossenen Nachweisverfahren können auf folgenden Gebieten zum Einsatz kommen:

Der Nachweis der Tumorzellen kann direkt als Tumormarker eingesetzt werden.

In Staginguntersuchungen kann die Anzahl der nachgewiesenen zirkulierenden Tumorzellen mit dem klinischen Bild korreliert und ein individuelles Tumorstaging festgelegt werden. Nach Entfernung des Primärtumors kann der Patient regelmäßigen Rezidivkontrollen unterzogen und bei positivem Befund sofort behandelt werden. Weitere Anwendungsmöglichkeiten sind der Nachweis von residualen Tumorzellen im Knochenmark von Patienten, die sich einer Hochdosis-Strahlentherapie unterziehen müssen oder von zirkulierenden Tumorzellen im Rahmen von ex-vivo und in-vivo Gentherapieansätzen.

Durch die Gewinnung von zirkulierenden Tumorzellen können Therapien auf ihre Wirksamkeit hin überprüft und gegebenenfalls abgeändert werden. Dies ist insbesondere dadurch möglich, daß man nach Gewinnung von Tumorzellen, direkt oder nach Kultur und Expansion, die individuelle Resistenzlage der Tumorzellen auf ein Zytostatikum überprüfen und alternative Präparate austesten kann. Außerdem besteht die Möglichkeit an den gewonnenen Tumorzellen neue Therapeutika zu testen.

Einen besonderen Stellenwert wird dem Verfahren im Rahmen von Tumorvorsorgeuntersuchungen beigemessen, da mit einer erheblich früheren Diagnose und bei sofortiger Therapie mit längeren Überlebensraten zu rechnen ist. Weitere Anwendungen liegen in der Tumorvakzineherstellung und der Gentherapie.

Die vorliegende Erfindung ermöglicht auch ein Verfahren zum Nachweis von Tumorzellen in einer Körperflüssigkeit, worin die Tumorzellen, wie vorstehend beschrieben aus der Körperflüssigkeit angereichert und bevorzugt gleichzeitig unerwünschte Zellen abgereichert werden.

In der immunzytologischen/immunzytochemischen Diagnostik werden z.B. zirkulierende Tumorzellen in Blut und Knochenmark durch spezifische Antikörper nachgewiesen. Dazu werden ca. 1 - 2 x $10^6$ mononukleäre Zellen (MNC) mittels einer Zentrifuge auf einen Objektträger gebracht, gefärbt und mikroskopisch ausgewertet (Zhong et al. Tumordiagn. u. Ther. (1999) 20: 39). Eine Blutprobe enthält zwischen 1 - 3 x $10^6$ MNC/ml. Ausgehend von einer mittleren Menge von 2 x $10^6$ MNC/ml, enthalten 20 ml Blut ca. 40 x $10^6$ MNC, die auf 20 - 40 Objektträgern untersucht werden müßten. Unsere Untersuchungen zeigen, daß mit dem erfindungsgemäßen Verfahren aus 20 ml Blut ca. 1 x $10^5$ Zellen angereichert werden können. Das bedeuted, daß die Auswertung der gewonnenen Tumorzellfraktion aus bis zu 200 ml Blut oder Knochenmark auf einem Präparat erfolgen kann.

An diesem Beispiel wird verdeutlicht, daß das erfindungsgemäße Verfahren entscheidende Bedeutung besonders in Hinblick auf folgende Faktoren hat: 1. Wirtschaftlichkeit: Einsparung von Zeit und Reagentien, 2. Qualität der Ergebnisse: z.B. durch Erhöhung der Signal/Noise-Ratio, 3. Erschließung bisher nicht durchführbarer zell- oder molekularbiologischer Untersuchungen der zirkulierenden Tumorzellen: z.B. Einzel-Zell-PCR-, FISH- und Gene-Array-Scanner-Analysen und 4. Automatisierung und Miniaturisierung der Nachweisverfahren: z.B. HTS (High Throuput Systeme) und die Nanotechnologie.

Nachdem im Rahmen einer manifesten Mikrometastasierung die Wahrscheinlichkeit einen positiven Befund zu erheben, signifikant verbessert wird, bietet das erfindungsgemäße Verfahren zudem entscheidende Vorteile in Bezug auf eine Standardisierung der Detektion von Tumorzellen sowohl im Knochenmark als auch im peripheren Blut, wie sie z.B. von der ISHAGE (International Society for Hematotherapy and Graft Engeneering) Working Group for Standardization of Tumor Cell Detection bei Borgen et al. in Cytotherapy (1999) 1: 377 gefordert wird.

Wie erwähnt, können einzelne Schritte oder mehrere Einzelschritte innerhalb des erfindungsgemäßen Anreicherungsverfahrens oder das ganze Verfahren selbst automatisiert werden. Für eine Automatisierung des Verfahrens eignen sich sämtliche technischen Verfahren in welchen die notwendigen Manipulation automatisch durch Roboter bzw. eigens konzipierte Maschinen unter standardisierten Bedingungen durchgeführt werden können. Weiterhin eignen sich Systeme in welchen die einzelnen Reaktionsschritte in minimalen Volumina durchgeführt werden können. Beispiele hierfür sind High Throuput Systeme (HTS) sowie Systeme der Nanotechnologie.

Beispielsweise erlaubt die Verwendung von Mikrotiterplatten einen höheren Automatisierungsgrad. Im Sinne des erfindungsgemäßen Anreicherungsverfahrens kann beispielsweise das gesamte Spektrum der gängigen Diagnostik wie z.B. mikroskopische, immunzytologische/immunzytochemische, biochemische und/oder molekularbiologische Verfahren auf Mikrotiterplattenebene durchgeführt werden. Mikrotiterplatten erlauben für den Fachmann die gleichen Manipulationen, mit dem Unterschied, daß mehrere Proben gleichzeitig, unter standardisierten Bedingungen und in schnelleren Zeiträumen verarbeitet werden können.

Im Sinne der Automatisierung des erfindungsgemäßen Verfahrens, können beispielsweise für den immunzytologischen/immunzytochemischen Nachweis, unterschiedliche Blut- oder Knochenmarkproben, nach einer erfolgten Auftrennung, getrennt zu einem Cytospin-Präparat verarbeitet oder alternativ direkt auf Mikrotiterplatten zentrifugiert werden. Ein Cytospin-Präparat hat eine Grundfläche von ca. 240 mm$^2$ auf die 1 x $10^6$ Zellen zentrifugiert werden. Als Mikrotiterplatten eignen sich beispielsweise 12 Kammer-, 24 Kammer- bzw. 48 Kammer-Platten mit einer Kammerfläche von jeweils ca. 350 mm$^2$, 190 mm$^2$ bzw. 110 mm$^2$.

Bevorzugt werden für die immunzytologische/immunzytochemische Diagnostik 12 Kammer- oder 24 Kammer-Platten und besonders bevorzugt 24 Kammer-Platten verwendet. Weiterhin werden bevorzugt Mikrotiterplatten verwendet, in denen die Kammern als Streifen in einen Rahmen eingesetzt werden können, z.B. 3 Streifen, à 4 Kammern/12 Kammer-, 4 Streifen à 6 Kammern/24 Kammer- und 6 Streifen à 8 Kammern/48 Kammer-Platte.

Besonders bevorzugt werden Mikrotiterplatten, die statt eines Plastikbodens einen Glasboden haben, da sich Glas für verschiedene Anwendungen besser als Plastik eignet (beispielsweise zur Fixierung der Zellen). Analog zu den Cytospin-Präparaten können die Präparate in Mikrotiterplatten durch eine computergestützte Bildanalyse ausgewertet werden.

Analog zur den Verfahren im Bereich der Immunzytologie /Immunzytochemie können die molekularbiologischen Diagnoseverfahren ebenfalls auf Mikrotiterplatten durchgeführt werden, mit dem Unterschied, daß nach der Auftrennung der Zellen wesentlich kleinere Volumina verarbeitet werden und somit Mikrotiterplatten bis hin zu HTS- (High Throughput System) Mikrotiterplatten bzw. Systeme der Nanotechnologie verwendet werden und die Manipulationen durch Roboter bzw. eigens konzipierte Maschinen unter standardisierten Bedingungen in minimalen Volumina durchgeführt werden können.

Im Rahmen der Automatisierung des erfindungsgemäßen Verfahrens können Einsätze auf die Mikrotiterplatten gesetzt werden, die es erlauben, daß die Auftrennung der Zellen in kleineren Volumina, analog zu einem Zentrifugationsgefäß, direkt auf einer Mikrotiterplatte durchgeführt werden können.

Die vorliegende Erfindung ermöglicht auch Verfahren zum semi- oder vollautomatischen Nachweis von Tumorzellen in einer Körperflüssigkeit, worin die Tumorzellen wie vorstehend beschrieben aus einer Körperflüssigkeit angereichert wurden.

In einer speziellen Anwendung des erfindungsgemäßen Verfahrens können auch Tumorzellen aus Körperflüssigkeit isoliert und beispielsweise zu Forschungs- und Therapiezwecken kultiviert werden. Zur Kultur eignen sich sämtliche Systeme wie auch Systeme der Nanotechnologie. In diesen Systemen können die Kulturen von Tumorzellen unter optimierten Bedingungen in minimalen Volumina semi- bzw. vollautomatisch durchgeführt werden.

Die vorliegende Erfindung ermöglicht somit auch Verfahren zur Kultur von Tumorzellen, die unter Anwendung des erfindungsgemäßen Verfahrens gewonnen wurden.

In einer speziellen Anwendung des erfindungsgemäßen Verfahrens können auch Tumorzellen beispielsweise aus dem Knochenmark bzw. aus dem peripheren Blut abgereichert werden, z.B. wenn der Spender zum Zwecke der Erhöhung des Blutstammzell-Gehaltes mit Wachstumsfaktoren behandelt wurde.

Eine Anreicherung von Blutstammzellen wird routinemäßig sowohl aus dem Blut als auch aus dem Knochenmark zum Zwecke der Blutstammzelltransplantation durchgeführt. Dabei kann es sich sowohl um eigene (autologe) oder fremde (allogene) Blutstammzellen handeln. Die autologe wie auch allogene Blutstammzelltransplantation wird besonders im Rahmen der Hochdosistherapie (z. B. Chemo- bzw. Strahlentherapie) von Tumorerkrankungen als auch zur Therapie von Erkrankungen des blutbildenden Systems und von Autoimmunerkrankungen (z.B. Rheuma) angewendet. Im Falle einer Anreicherung von Blutstammzellen aus dem Knochenmark, wird das Ausgangsmaterial direkt durch Entnahme von Knochenmark, z. B. aus dem Beckenkamm gewonnen. Im Falle einer Anreicherung von Blutstammzellen aus dem peripheren Blut wird die Konzentration der Blutstammzellen im Blut zuerst durch die Gabe von Wachstumsfaktoren erhöht. Mononukleäre Zellen (MNC) werden nachfolgend mittels Leukapherese gewonnen. Die gewonnenen mononukleären Zellen werden dann einem Anreicherungsverfahren für Blutstammzellen unterzogen. Diese angereicherte Blutzell-Fraktion wird dann mit DMSO versetzt und bis zur Transplantation eingefroren.

In Falle der autologen Blutstammzelltransplantation ist die Gefahr der Transplantation von kontaminierenden Tumorzellen besonders groß und gefährdet den Therapieerfolg. Es ist deshalb wünschenswert vor der Transplantation die gewonnene MNC-Fraktion auf das Vorhandensein von Tumorzellen zu überprüfen und vorhandene Tumorzellen abzureichern.

Es wurde nun gefunden, daß Blutstammzellen, die als Oberflächenmarker das CD34 Antigen tragen, Telomerase-positiv sind, und eine Dichte von ca. 1,061 g/ml $\pm$ 0,0005 g/ml haben. Diese Dichte liegt so nahe an der besonders bevorzugten Dichte von 1,060 g/ml $\pm$ 0,0005 g/ml, die im erfindungsgemäßen Verfahren zur Anreicherung von Tumorzellen verwendet wird, daß eine perfekte Trennung von Telomerase-positiven Tumorzellen von Telomerase-positiven Nicht-Tumorzellen, nach einer therapeutischen Mobilisierung von CD 34+ Blutstammzellen in das periphere Blut, nicht immer vollständig und sicher gewährleistet werden kann.

Im Falle des Telomerase-Nachweises aus dem peripheren Blut, belegen durchgeführte Versuche, daß bei allen Blutproben der untersuchten Probanden, selbst nach einer Auftrennung mit einem Zellseparationsmedium mit einer Dichte von 1,065 g/ml, die aus der Interphase gesammelte Zellfraktion durchgehend Telomerase-negativ war. Das bedeutet, daß bei den untersuchten Probanden der Anteil von CD 34+ Zellen, bzw. die Telomerase-Aktivität der CD 34+ Zellen im peripheren Blut unter der Nachweisgrenze lag (Beispiel 2).

Nach der Mobilisierung von Blutstammzellen in das periphere Blut ist es wahrscheinlich, daß die gesammelte Zellfraktion bei einer Trennung mit einer Dichte von 1,060 $\pm$ 0,0005 g/ml, Telomerase-positiv wird und nicht mehr zwischen Telomerase-positiven Tumorzellen und Telomerase-positiven Nicht-Tumorzellen unterscheiden werden kann. Dies liegt darin begründet, daß durch die Stimulation mit den Wachstumsfaktoren zum einen die Menge der CD 34+ Blutstammzellen und zum anderen auch die Telomerase-Aktivität dieser Zellen zugenommen hat. Ein Teil dieser CD 34+ Blutstammzellen wird nicht vollständig abgereichert und somit werden die in der Interphase befindlichen Telomerase-positiven Tumorzellen durch Telomerase-positive CD 34+ Nicht-Tumorzellen kontaminiert.

Dieses Problem kann erfindungsgemäß dadurch gelöst werden, daß in diesem Falle das vorstehend beschriebene Verfahren dahingehend abgewandelt wird, in einem ersten Schritt die CD 34+ Blutstammzellen anzureichern und die unerwünschten Blutzellen in einem hohen Maße abzureichern. In einem zweiten Schritt werden dann entweder die Tumorzellen von den CD 34+ Blutstammzellen oder die CD 34+ Blutstammzellen von den Tumorzellen durch Immunadsorption abgetrennt. Es wurde gefunden, daß eine besonders gute Trennleistung, im Sinne einer Abreicherung von unerwünschten Blutzellen bei gleichzeitiger Anreicherung von CD34+ positiven Blutstammzellen und Tumorzellen mit einer Erhöhung der Dichte des Zellseparationsmediums auf einen Bereich von 1,061 bis 1,065 g/ml und besonders bevorzugt von etwa 1,062 g/ml, insbesondere 1,062 g/ml $\pm$ 0,0005 g/ml erzielt wird.

Der zweite Trennschritt erfolg durch eine weitere Anreicherung der gewünschten Zellpopulationen bzw. Abreicherung unerwünschter Zellen wie z.B. Tumorzellen in Blutstammzell-Präparationen zu diagnostischen wie therapeutischen Zwecken, und wird durch ein nachgeschaltetes An- oder Abreicherungsverfahren z.B. unter Verwendung von Immunadsorptionsverfahren mit spezifischen Antikörpern durchgeführt.

Durch das erfindungsgemäße Trennverfahren kann ein nachgeschaltetes Abreicherungsverfahren wesentlich kostengünstiger angewendet werden, nachdem ein erheblicher Anteil der unerwünschten Blutzellen bereits abgereichert worden ist.

Die vorliegende Erfindung ermöglicht auch ein Verfahren insbesondere zum Nachweis und zur therapeutischen Abreicherung von Tumorzellen aus Blutstammzellen von Knochenmark und peripherem Blut, worin die Tumorzellen und Blutstammzellen in einer Fraktion wie vorstehend beschrieben angereichert und die Blutstammzellen oder Tumorzellen in einem zweiten Schritt entweder angereichert oder abgereichert werden.

Nachdem das erfindungsgemäße Verfahren vorteilhaft im Rahmen der therapeutischen autologen Blutstammzellgewinnung eingesetzt werden kann, ist es naheliegend, dieses Verfahren auch zur allogenen Blutstammzellgewinnung einzusetzen.

Das erfindungsgemäße Anreicherungsverfahren von allogenen und autologen Blutstammzellen bewirkt, daß die Blutstammzellen erheblich besser angereichert und unerwünschte Blutzellen wesentlich besser abgereichert werden, als es mit den bisher üblichen Methoden durchführbar ist. Dies hat insbesondere den Vorteil, daß die Menge von DMSO, die zur Kryokonservierung der Zellen erforderlich ist, erheblich verringert werden kann. Somit können die durch DMSO verursachten Komplikationen, die bei der Blutstammzelltransplantation entstehen, verringert werden.

Die vorliegende Erfindung ermöglicht somit auch ein Verfahren zur therapeutischen Anreicherung von allogenen bzw. autologen Blutstammzellen von Knochenmark und peripherem Blut, worin bei der autologen Blutstammzellgewinnung die Tumorzellen und Blutstammzellen in einer Fraktion wie vorstehend beschrieben angereichert und die Blutstammzellen oder Tumorzellen in einem zweiten Schritt entweder angereichert oder abgereichert werden.

Ein weiterer Gegenstand der vorliegenden Erfindung ist ein Zentrifugations gefäß nach Anspruch 18 zur Anreicherung von Tumorzellen aus einer Körperflüssigkeit, der zur Durchführung des erfindungsgemäßen Verfahrens geeignet ist. Hierzu umfaßt der Kit ein Zellseparationsmedium, das eine Dichte im Bereich von 1,059 bis 1,065 g/ml aufweist, bevorzugt im Bereich von 1,059 bis 1,063 g/ml, bevorzugter von 1,059 bis 1,061 g/ml und besonders bevorzugt von etwa 1,060 g/ml, insbesondere 1,060 g/ml ± 0,0005 g/ml sowie gegebenenfalls ein Zentrifugationsgefäß.

Der Kit dient auch der Anreicherung von Blutstammzellen aus peripherem Blut und dem Knochenmark. Hierzu umfaßt der Kit ein Zellseparationsmedium, das eine Dichte im Bereich von 1,061 bis 1,062 g/ml, insbesondere 1,062 g/ml ± 0,0005 g/ml aufweist sowie gegebenenfalls ein Zentrifugationsgefäß.

Um das routinemäßige Arbeiten mit dem Kit zu erleichtern, weist das Zentrifugationsgefäß eine poröse Barriere bevorzugt mit einer Dicke von 1-10 mm, vorzugsweise ca. 1-5 mm auf, die das Zentrifugationsgefäß in ein oberes und ein unteres Kompartiment unterteilt. Die poröse Barriere weist
eine Porengröße von 20-100 $\mu$m, vorzugsweise 20-30 $\mu$m auf und besteht bevorzugt aus einem hydrophoben Material.

Die Größe des in dem Kit enthaltenen Zentrifugationsgefäßes sollte an die Menge der Körperflüssigkeit angepaßt sein, aus der die Tumorzellen angereichert werden sollen. Beispielsweise kann das Zentrifugationsgefäß ein Volumen von 1-500 ml, bevorzugt 1-50 ml und besonders bevorzugt 15-50 ml aufweisen. Bevorzugt ist das Zentrifugationsgefäß verschließbar. Das Zentrifugationsgefäß ist bevorzugt steril bzw. sterilisierbar und kann dabei aus festen unverformbaren oder auch verformbaren Materialien (Beutel) bestehen oder eine Mikrotiterplatte sein.

In einer alternativen Ausführungsform sollte die Größe des in dem Kit enthaltenen Zentrifugationsgefäßes an die Menge der Körperflüssigkeit angepaßt sein, aus der die Blutstammzellen angereichert werden sollen. Beispielsweise kann das Zentrifugationsgefäß ein Volumen von 50-500 ml, bevorzugt 50-250 ml und besonders bevorzugt 50-200 ml aufweisen. Bevorzugt ist das Zentrifugationsgefäß verschließbar. Das Zentrifugationsgefäß ist steril bzw. sterilisierbar und kann dabei aus festen unverformbaren oder auch verformbaren Materialien (Beutel) bestehen.

Besonders vorteilhaft befindet sich das Zellseparationsmedium in dem Kit bereits im unteren Kompartiment des Zentrifugationsgefäßes, damit dieses bei Routineuntersuchungen einfach und schnell eingesetzt werden kann.

In einer weiteren bevorzugten Ausführungsform umfaßt der Kit ein Zellseparationsmedium, das mit einem Farbstoff versetzt ist, der die Interphase zwischen Zellseparationsmedium und Interphase nach der Zentrifugation leichter sichtbar macht.

Das erfindungsgemäße Verfahren bietet den Vorteil, daß Telomerase-positive nicht-Tumorzellen einfach und sicher von den anzureichernden Tumorzellen abgetrennt werden, so daß in anschließenden Nachweisverfahren keine falsch-positiven Ergebnisse durch Telomerase-aktive nicht-Tumorzellen erhalten werden. Darüber hinaus sind nur wenige Arbeitsschritte für die Anreicherung und Isolierung von Tumorzellen aus Körpergewebe notwendig, so daß dadurch die Prozessierung von größeren Mengen von Probenmaterial möglich wird. Die Kosten für die notwendigen Materialien sind beispielsweise gegenüber der Verwendung spezifischer Antikörper und der anschließenden Trennung mittels geeigneter Apparaturen signifikant geringer.

Darüber hinaus zeigte die Untersuchung von 10 unterschiedlichen Zellinien, die von Tumorgeweben, wie Melanom, Prostata-, Brust-, Lungen-, Leber- und Colorektal-Karzinomen abgeleitet waren, daß die Zellen all dieser Zellinien in ihrer Mehrzahl durch das erfindungsgemäße Verfahren angereichert wurden.

Von den anliegenden Figuren zeigt:

Figur 1 das Ergebnis einer Untersuchung der Temperaturabhängigkeit der Dichte von Percoll. In einem Temperaturbereich von ca. 0°C-8°C beträgt die Dichte der hergestellten Percoll-Arbeitslösung 1,060 ± 0,005 g/ml. Bei einer Umgebungstemperatur von mehr als 8°C beginnt die Percoll-Arbeitslösung stetig zu expandieren und die ursprünglich eingestellte Dichte signifikant abzunehmen.

Figur 2 zeigt das Ergebnis einer RT-PCR-Analyse von Blut eines gesunden Spenders (A) und Blut des gleichen Spenders gemischt mit GFP-transfizierten Zellen der Melanom-Zelllinie T289 (B, C) nach Tumorzellanreicherung mit einem Zellseparationsmedium einer Dichte von 1,070 g/ml.

Figur 3 zeigt das Ergebnis einer RT-PCR-Analyse von Blut gesunder Spender, das mit Tumorzellen einer Prostata-Karzinom-(A) und Mamma-Karzinom-Zelllinie (B) gemischt wurde, nach Tumorzellanreicherung mit einem Zellseparationsmedium einer Dichte von 1,065 g/ml.

Figur 4 zeigt die Wiederfindungsrate von GFP-transfizierten Melanomzellen, die zu Blut unterschiedlicher (A, B) gesunder Spender gemischt wurden, nach Anreicherung mit einem Zellseparationsmedium einer Dichte von 1,065 g/ml.

Figur 5 zeigt ein Flußdiagramm für die RT-PCR.

Figur 6 zeigt die Wiederfindungsrate gespikter Tumorzellen der Brustkrebs-Zell-Linie MDMB 435s im peripheren Blut.

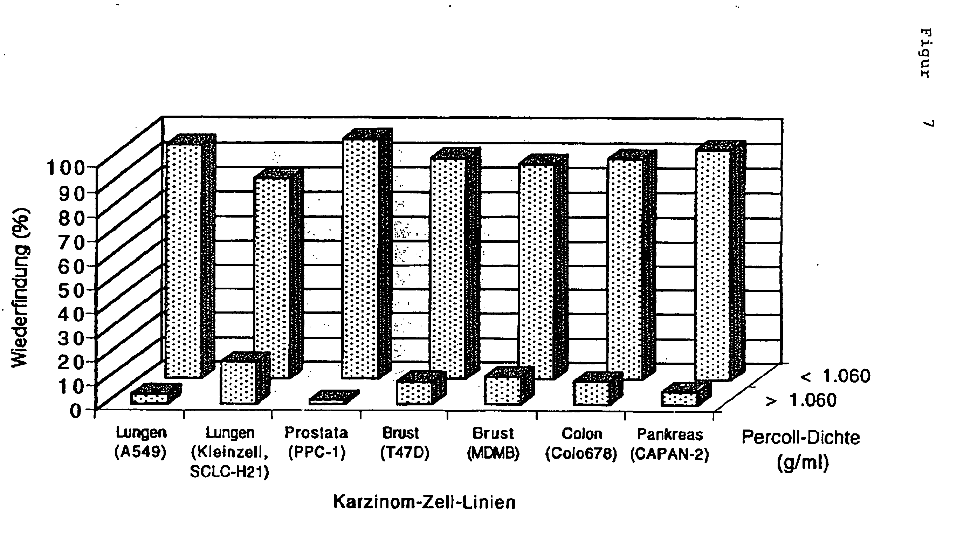

Figur 7 zeigt die Wiederfindungsrate von unterschiedlichen Karzinomzell-Linien.

Die nachfolgenden Beispiele sollen die Erfindung näher erläutern.

Beispiel 1

In einem silikonisierten Kunststoff-Zentrifugationsgefäß wurde venöses Blut (5-20 ml), mit EDTA versetzt (3,9 mM Endkonzentration, pH 8,0) und mit 1 Volumen PBS gemischt. Das Blut/PBS-Gemisch wurde anschließend auf 5-10 ml Percoll mit einer Dichte von 1,065 g/ml gegeben und bei langsamer Beschleunigung und ohne Bremse für 30 Minuten bei 1.000 x g und 4°C zentrifugiert. Das untere Viertel des Zentrifugationsgefäßes wurde anschließend für 5-10 min in flüssigem Stickstoff inkubiert. Dadurch wurde während des Absaugens der Zellen, die sich an der Interphase im Übergang zwischen dem Percoll und dem darüberliegenden Plasma/PBS-Gemisch befanden, eine Kontamination mit Zellen des Pellets verhindert. Die Zellen der Interphase, bei denen es sich vorwiegend um Thrombozyten und um im Blut zirkulierende Tumorzellen handelte, wurden anschließend in ein neues silikonisiertes Kunststoff-Zentrifugationsgefäß übertragen und für 10 min bei 1.000 x g und 4°C zentrifugiert. Für die anschließende RT-PCR-Untersuchung wurde das Zellpellet in einem Guanidium-Isothiocyanat-Puffer aufgenommen, wodurch die Zellen lysiert wurden und einer RNA-Isolierung unterzogen werden konnten.

Beispiel 2

Mit Hilfe von sogenannten Spiking-Experimenten, bei denen Tumorzellen verschiedener Zelllinien zum Blut normaler Spender gemischt wurden und die Tumorzellen anschließend re-isoliert und in der RT-PCR untersucht wurden, wurde gezeigt, daß in Abhängigkeit der verwendeten Zelllinie die Telomerase-Aktivität von etwa 1-4 gespikter Tumorzellen/ml Blut nachgewiesen werden kann. Die RT-PCR wurde analog zu der in Beispiel 4 beschriebenen Vorgehensweise durchgeführt.

Hierzu wurden die Zellen der zu spikenden Tumorzelllinien entsprechend der Angaben des Herstellers (ATCC, *American Tissue Cell Culture*) bis zur Konfluenz kultiviert. Die Zellen wurden anschließend trypsiniert und in Medium (RPMI 1640) gewaschen. Nach Entnahme eines 10 μl Aliquots, das 1:1 mit Tryptan-Blau gemischt wurde, wurden die lebenden Zellen in einer Zählkammer bestimmt und die entsprechende Zellkonzentration wurde berechnet. Anschließend wurde die Zellsuspension verdünnt und ein Volumen, das einer bestimmten Zellzahl entspricht, mit dem Blut gesunder Blutspender gemischt. Als Kontrolle diente Blut dem keine Tumorzellen zugesetzt wurden. Die Anreicherung der gespikten Tumorzellen wurde einmal zum Vergleich mit einem Zellseparationsmedium einer Dichte von 1,070 g/ml und nach dem erfindungsgemäßen Verfahren durchgeführt. Zur Bestimmung der Wiederfindungsrate wurden anschließend mikroskopische, durchflußzytometrische und RT-PCR-Analysen durchgeführt.

a) Vergleichsversuch

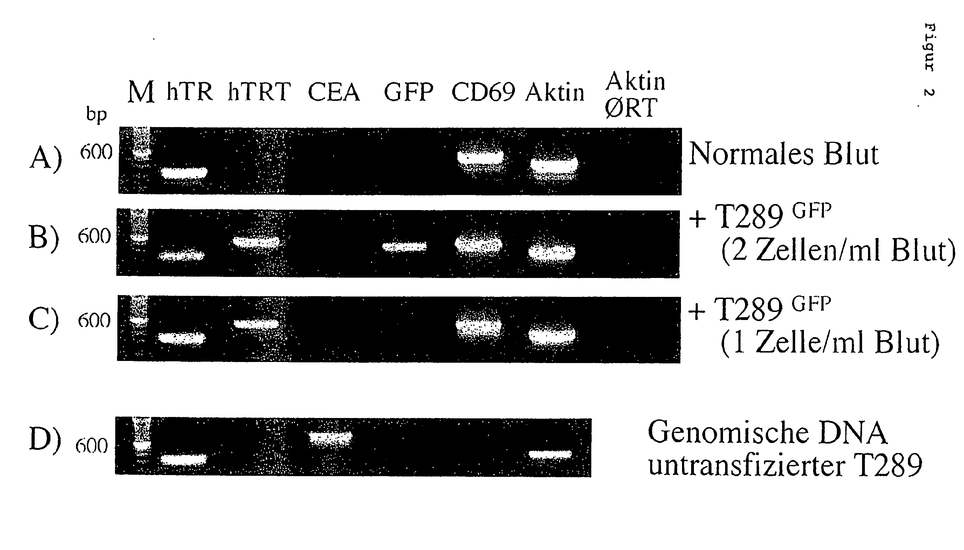

Figur 2 zeigt das Ergebnis einer RT-PCR-Analyse von 20 ml Blut eines gesunden Spenders (A) und 20 ml Blut des gleichen Spenders gemischt mit GFP-transfizierten Zellen der Melanom-Zelllinie T289 (B, C). Das Blut wurde auf Percoll einer Dichte von 1,070 g/ml geschichtet, zentrifugiert und anschließend wurden die Zellen analysiert. Die katalytische Untereinheit der Telomerase (hTRT) ist im normalen Blut nicht nachweisbar (A), während bei 1 und 2 gespikten Melanomzellen pro ml Blut hTRT nachweisbar ist (B, C). Bei der verwendeten Percoll-Dichte von 1,070 g/ml sind jedoch noch hinreichend viele Telomerase-aktive Leukozyten in der Interphase vorhanden, wodurch die RNA-Komponente (hTR) auch im ungespikten Blut nachweisbar ist. Für die Präsenz von aktivierten und wahrscheinlich deshalb auch Telomerase-aktiven Leukozyten in der Fraktion der isolierten Zellen spricht auch die Tatsache, daß CD69, ein früher Aktivierungsmarker in B- und T-Zellen, in allen Blutproben nachweisbar ist (A-C). Der Tumormarker CEA (Carcinoembrionic Antigene) ist sowohl im ungespikten als auch im gespikten Blut negativ (A-C). GFP (Green Fluorescent Protein), der als zusätzlicher Marker für die gespikten Tumorzellen verwendet wurde, ist im ungespikten Blut nicht nachweisbar (A). Da nur etwa 50% der transfizierten T289-Melanomzellen GFP exprimieren, ist das Protein nur in bis zu 2 gespikten Tumorzellen pro ml Blut nachweisbar (B). Aktin diente als RT-PCR-Positivkontrolle (Aktin) und im Ansatz ohne RT-Reaktion als Negativkontrolle (Aktin ØRT). Die PCR-Amplifikation genomischer DNA untransfizierter T289-Zellen führt mit den spezifischen Primerpaaren für hTRT, GFP und CD69 zu keinen Amplifikaten.

b) erfindungsgemäßer Versuch

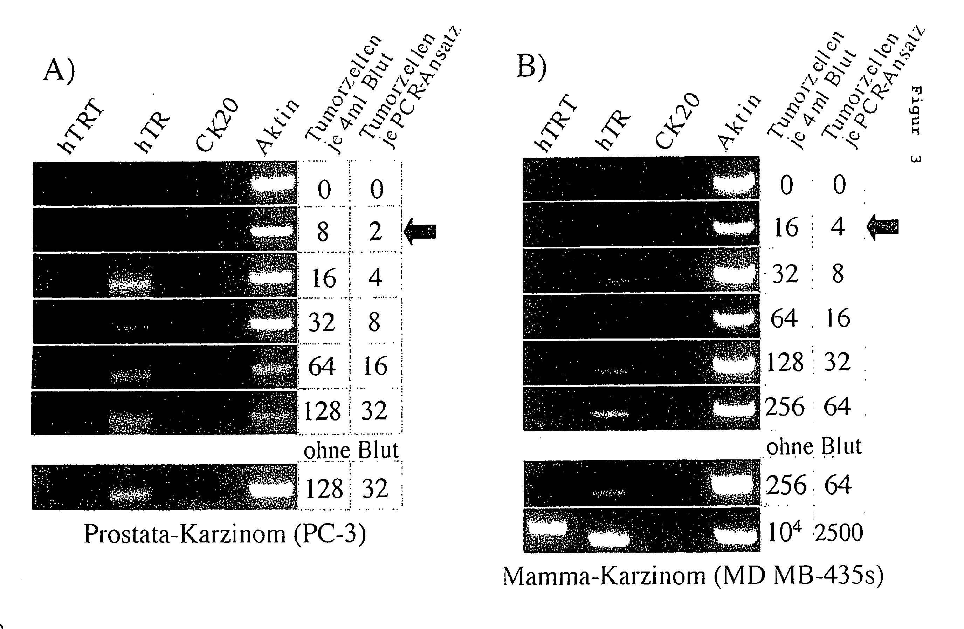

Figur 3 zeigt RT-PCR-Analysen von Blut gesunder Spender das mit Tumorzellen einer Prostata-Karzinom- (A) und Mamma-Karzinom-Zellinie (B) gemischt, auf Percoll einer Dichte von 1,065 g/ml geschichtet, zentrifugiert und anschließend analysiert wurde. Die RNA-Komponente der Telomerase (hTR) ist im Unterschied zu der Verwendung von Percoll mit einer Dichte von 1,070 g/ml im ungespikten Blut nicht nachweisbar (vgl. Fig. 1). In den Proben mit 2 gespikten Prostata-Karzinomzellen (A) bzw. mit 4 gespikten Mamma-Karzinomzellen (B) pro ml Blut kann hTR nachgewiesen werden (schwarzer Pfeil). Im Unterschied zur Melanom-Zellinie T289 konnte bei diesen Tumorzellen keine (A) bzw. erst bei $10^4$ Tumorzellen (B) eine Expression der katalytischen Untereinheit (hTRT) nachgewiesen werden. Weder der Prostatazell-spezifische Marker PSA (Prostate Specific Antigene) noch der Epithelzell-spezifische Marker CK20 (Cytokeratin 20) ist in den entsprechenden Tumorzellen nachweisbar. Aktin dient als RT-PCR-Positivkontrolle.

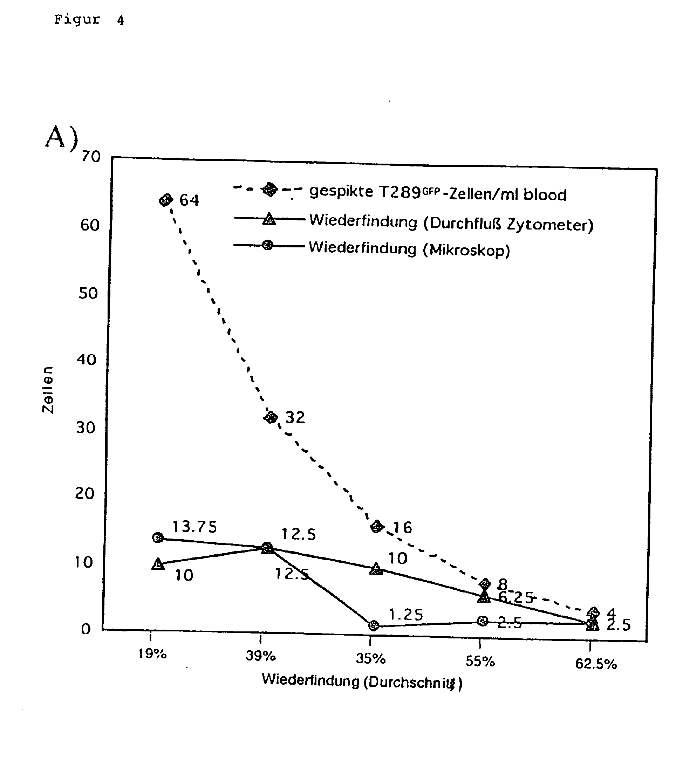

Figur 4 zeigt die Wiederfindungsraten von GFP-transfizierten Melanomzellen (T289), die zu Blutproben gesunder Spender gemischt (gespikt) wurden. Die gespikten Blutproben wurden anschließend auf Percoll einer Dichte von 1,065 g/ml geschichtet, zentrifugiert und die Anzahl der re-isolierten Tumorzellen (Wiederfindung) wurde mikroskopisch (-●-) und/oder durchflußzytometrisch (-▲-) bestimmt. Da nur etwa 75% für Probe A bzw. 50% für Probe B der GFP-transfizierten T289-Zellen im Durchflußzytometer nachweisbar waren, wurden die Wiederfindungsraten entsprechend korrigiert. Die Wiederfindungsrate gespikter Tumorzellen ist abhängig vom jeweiligen Blutspender (die Blutproben von A) und B) stammen von unterschiedlichen Spendern), der verwendeten Zellinie und verhält sich umgekehrt proportional zur Anzahl der gespikten Tumorzellen. Möglicherweise führt eine Abstoßungs-Reaktion der entsprechenden hämatopoetischen Zellen zur Lyse, Aggregation und schließlich zum Verlust der gespikten allogenen Tumorzellen. B) zeigt darüber hinaus, daß die Anzahl der tatsächlich gespikten Tumorzellen (-■-) zwischen 6% - 37% geringer ist als die theoretisch berechnete Anzahl gespikter Tumorzellen (-♦-).

Unter Hinzunahme hier nicht dargestellter Untersuchungen mit Lungen- und Mamma-Karzinomzellen ergibt sich eine durchschnittliche Wiederfindungsrate mit der erfindungsgemäßen Anreicherungsmethode von 46% $\pm$ 20% für 4-512 gespikte Zellen (n=16) und für $\leq$ 50 gespikte Zellen von 54% $\pm$ 20% (n=15).

Damit liegt die Wiederfindungsrate des erfindungsgemäßen Tumorzellanreicherungsverfahrens etwa im Bereich von magnetischen Zell-Separatoren wie MACS für die eine Wiederfindungsrate von etwa 30-58% angegeben wird.

Beispiel 3

Erste klinische Untersuchungen bei Melanom-Patienten haben gezeigt, daß in 43% der Patienten mit akuten Metastasen und in 16% der Patienten ohne offenkundige akute Tumorerkrankung (beispielsweise nach Resektion der Tumoren bzw. nach Therapie) die Telomerase in den mit dem erfindungsgemäßen Verfahren angereicherten disseminierten zirkulierenden Tumorzellen des Blutes nachgewiesen werden konnte. Parallel untersuchte Blutproben von 10 gesunden Spendern waren dagegen negativ.

Damit zeigte bereits diese Studie an Melanom-Patienten eine eindeutige Korrelation der Telomerase-Aktivität von disseminierten zirkulierenden Tumorzellen im Blut und dem Metastasen-Status der entsprechenden Tumorpatienten.

Beispiel 4

4.1 Isolierung von zellulärer RNA

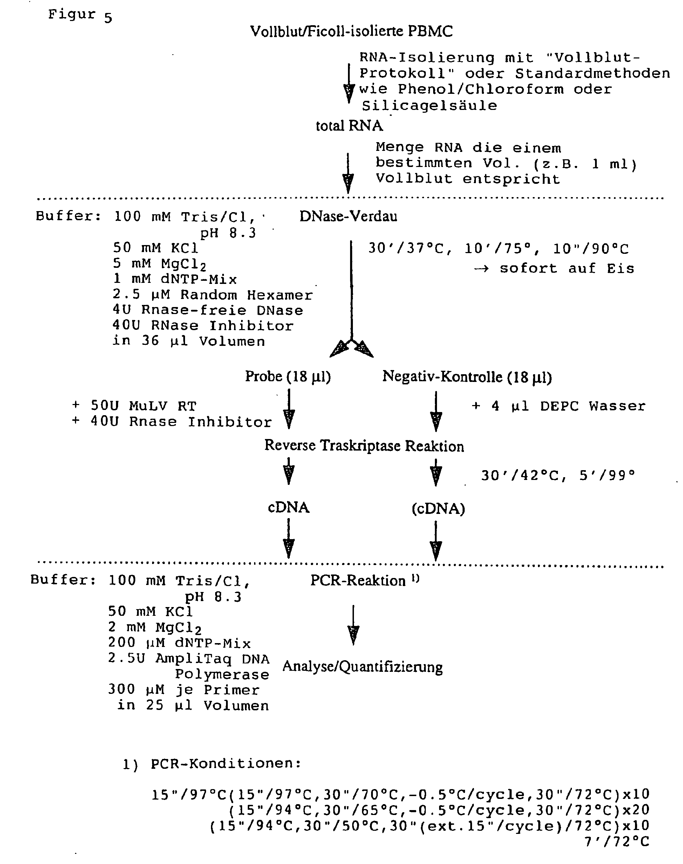

Die Isolierung von totaler zellulärer RNA erfolgte nach Standardverfahren [Chomczynski et al. (1987) Anal Biochem 162, 156; Sambrook, J. et al. (1989). Cold Spring Harbor, New York, USA, Cold Spring Harbor Laboratory Press]. Peripheres Blut wurde wie in Figur 5 gezeigt sofort nach Abnahme in RNA-Lysispuffer (4 M Guanidinium Isothiocyanat; 0.1 M Tris-HCl, pH 7.5; 1% Mercaptoethanol) überführt und homogenisiert. Die Gemische wurden entweder sofort weiterverarbeitet oder bei -70°C gelagert.

4.2 Reverse Transkription und Polymerase-Kettenreaktion (RT-PCR)

Die RT-PCR wurde mit dem GeneAmp® RNA-PCR-Kit (Perkin Elmer) nach Vorgaben des Herstellers wie in Figur 5 gezeigt durchgeführt. Aliquote der isolierten totalen RNA von peripherem Blut und Zelllinien wurden jeweils zuvor mit 4U DNAse und 40U RNAse Inhibitor (Boehringer, Mannheim) in 36 $\mu$l Ansätzen (in 100 mM Tris-HCl, pH 8.3; 50 mM KCl; 5 mM MgCl$_2$, 1mM dNTP-Mix und 2,5 mM Random Hexamers) bei 37°C für 30 Minuten und bei 75°C für 10 Minuten hydrolysiert und anschließend die DNAse für 10 Minuten bei 90°C inaktiviert und dann das Reaktionsgemisch sofort auf Eis gegeben.
Die beiden Oligonukleotid-Primer:

5' CTACCGGAAG AGTGTCTGGA GCAAGTTGCA AAGC 3' (hTRT1) und
5' GGCATACCGA CGCACGCAGT ACGTGTTCTG 3' (hTRT2)

wurden nach der von Nakamura et al. veröffentlichten Sequenz, kodierend für die katalytische Untereinheit der menschlichen Telomerase (Nakamura et al. (1997). Science 277: 955-9) entworfen und mit einem Applied Biosystem 380A Synthesizer synthetisiert. Die Spezifität der hTRT1- und hTRT2-Primer wurde mittels Computer gestützter Homologieanalyse an den Nukleinsäuresequenzen in den GenBank-, EMBL-, DDBJ- und PDB-Datenbanken mittels BLASTN 1.4.9 MP [Altschul, S. F. et al. (1990). J Mol Biol 215: 403-410] überprüft.
Zur Abstimmung der Amplifikationsmengen wurden für jedes Experiment gleiche RNA-Mengen für die RT-Reaktion eingesetzt. Um eine Kontamination der RNA Präparationen mit genomischer DNA auszuschließen, wurde jede mit DNase hydrolysierte RNA-haltige Probe zuerst der unten beschriebenen PCR unterzogen und auf Amplifikation hin überprüft. Die RNA-haltige Probe, bei der kein Amplifikationsprodukt nachweisbar war, wurde für die folgenden cDNA-Synthese- und PCR-Schritte eingesetzt. Als interne Standardkontrolle wurden Oligonukleotid-Primer für $\beta$-Aktin und den TCR eingesetzt. Die Reverse Transkriptase-Reaktion wurde an 18 $\mu$l des DNAse Verdaues unter Zugabe von 50U MuLV Reverser Transkriptase und 40U RNase Inhibitor bei 42°C für 30 Minuten durchgeführt und die Reaktion bei 99°C für 5 Minuten abgebrochen. In den Negativkontrollen wurden statt der Enzyme 4 $\mu$l Wasser zugesetzt.
Die PCR wurde wie in Figur 5 gezeigt an 5 $\mu$l der cDNA-Reaktion nach folgendem Programm durchgeführt: (97°C: 15 Sekunden vorwärmen); (97°C: 15 Sekunden, 70°C: 30 Sekunden [minus 0.5°C pro Zyklus], 72°C: 30 Sekunden) 10 Zyklen; (94°C: 15 Sekunden, 65°C: 30 Sekunden [minus 0.5°C pro Zyklus], 72°C: 30 Sekunden) 20 Zyklen; (94°C: 15 Sekunden, 50°C: 30 Sekunden 72°C: 30 Sekunden [plus 15 Sekunden Extension pro Zyklus], 10 Zyklen; (72°C: 7 Minuten, finale Extension).
Die Amplifikationsprodukte wurden gelelektrophoretisch auf 1.5%igem TAE Agarosegel aufgetrennt, mit Ethidiumbromid gefärbt und unter UV-Licht visualisiert und fotodokumentiert.

Beispiel 5

Es wurden weitere Spiking-Experimente, wie in Beispiel 1 und 2 beschrieben durchgeführt, mit dem Unterschied, daß normale nicht silikonisierte Polypropylen-Zentrifugationsgefäße und Percoll mit einer Dichte von 1,060 g/ml verwendet wurden. In diesen Experimenten sollte zum einen der Grad der Abreicherung von unerwünschten Blutzellen und zum anderen der Grad der Anreicherung von Tumorzellen bestimmt werden.
Bei ca. 80%-90% Konfluenz wurden die Tumorzellkulturen der Brustkrebs-Zell-Linie MDMB 435s trypsiniert und die gewonnene Zellsuspension in ein 15 ml Zentrifugationsgefäß überführt und diese bei 500 x g für 5 Minuten zentrifugiert. Das Zellpellet wurde in 0.5 ml resuspendiert.
100 ml der Zellsuspension (ca. $10^5$-$10^6$ Zellen) wurden zur Färbung des Zellkerns mit 20 ml einer DAPI-Lösung (interkalierender Farbstoff; 1 mg 4',6'-Diamidino-2-phenylindol Dihydrochlorid/ml Dimethylsulfoxyd [DMSO]) in einem 1,5 ml Zentrifugationsgefäß gemischt und für 10 Minuten bei 37°C und bei 700 rpm in einem Thermomixer (Eppendorf) inkubiert. Anschließend wurden die Zellen bei 500 x g für 5 Minuten pelletiert, in 1 ml DPBE (1 ml 0,1% BSA und 4 mM EDTA in Dulbecco PBS) resuspendiert und erneut bei 500 x g für 5 Minuten zentrifugiert. Der Waschschritt wurde zweimal wiederholt. Die Zellen wurden dann mit Hilfe eines PartikelCounters (Z2, Beckman Coulter GmbH) auf 2000 Zellen/ml DPBE eingestellt.
Triplikate von jeweils 10, 20, 30, 40, 50, 60 und 70 ml der DAPI-positiven Zellsuspension wurden individuell in die Kammern einer 384-Platte pipettiert. Die Mikrotiterplatte wurde dann bei 700 x g für 3 Minuten zentrifugiert und die Zellen mit Hilfe eines Fluoreszenzmikroskops (Axiovert 25, Zeiss, Filtersatz 02, [Extinktion 358 nm, Emission 460 nm]) ausgezählt. Danach wurde eine Standardgerade (x = ml DAPIpositive Zellsuspension; y = Zellzahl) gebildet. Die $r^2$-Werte der Standardgerade betrugen mindestens 0,95.
In den Spiking-Experimenten wurde in Triplikaten eine entsprechende Anzahl DAPI-positiver Zellen in 20 ml Vollblut (Bayerisches Rotes Kreuz, BRK) gemischt und das Vollblut in ein 50 ml Zentrifugationsgefäß mit einer porösen Barriere (Porengröße 20 - 100 mm) gegeben, in dessen unteren Kompartiment 15 ml Percoll mit einer Dichte von 1,060 g/ml (bei 4°C) und einer Osmolarität von 280-300 mmol/kg vorgelegt worden war. Anschließend wurde das Zentrifugationsgefäß bei 4°C und 1000 x g und 4°C für 30 Minuten zentrifugiert.

Nach der Zentrifugation wurden die Zellen der Interphase mit Hilfe einer 10 ml Pipette in ein frisches 50 ml Zentrifugationsgefäß überführt. Das obere Kompartiment des ersten Zentrifugationsgefäßes wurde zweimal vorsichtig mit 15 ml DPBE ausgewaschen und die Flüssigkeit in das zweite Zentrifugationsgefäß überführt. Anschließend wurde das Zentrifugationsgefäß auf 50 ml mit DPBE aufgefüllt und bei 200 x g für 10 Minuten zentrifugiert. Nach der Zentrifugation wurde der Überstand dekantiert und das Zellpellet in 5 ml Erythrozyten-Lysispuffer (155 mM $NH_4Cl$, 10 mM $KHCO_3$ und 0,1 mM EDTA pH 7,3) resuspendiert und bei Raumtemperatur für 5 Minuten inkubiert. Nach Beendigung der Erythrozytenlyse, wurde das Zentrifugationsgefäß auf 50 ml mit DPBE aufgefüllt und erneut bei 200 x g für 10 Minuten zentrifugiert und der Überstand dekantiert. Die Zellen wurden dann erneut in 50 - 200 ml DPBE aufgenommen.

Anschließend wurde die Zellsuspension in zwei gleiche Aliquote aufgeteilt. Ein Aliquot wurde in die Kammer einer 24-Platte gegeben und die Anzahl der Tumorzellen in der Mikrotiterplatte unter dem Mikroskop bestimmt. Die Gesamtmenge an Zellen des zweiten Aliquots wurde mit einem automatischen Haematologie-Analysesystem (KX21, Sysmex) bestimmt.

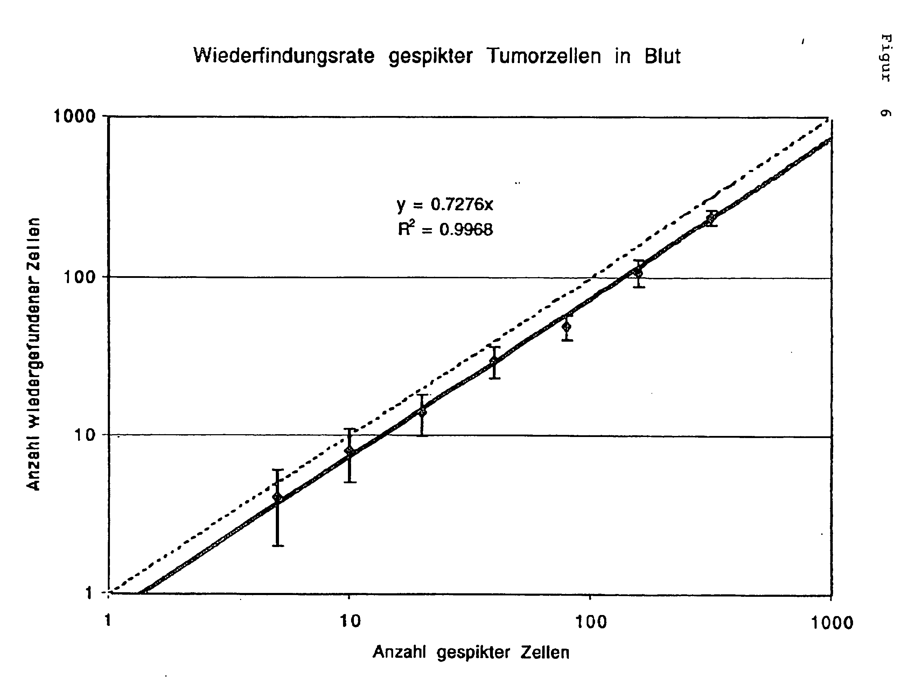

Die Experimente mit einem Percoll-Zellseparationsmedium mit einer Dichte von 1,060 g/ml, zeigen, 1. eine Wiederfindungsrate von ca. 73%, für die gespikten Tumorzellen (5 - 320 gespikte Zellen, Figur 6) und 2. einen Abreicherungsfaktor von ca. $10^3$ für Leukozyten.

Beispiel 6

Mit der Fragestellung, ob mit dem erfindungsgemäßen Verfahren auch weitere Tumorzell-Linien angereichert werden können, wurden folgende Untersuchungen durchgeführt: Tumorzell-Linien wurden entsprechend der Angaben von ATCC kultiviert und, wie unter Beispiel 2 und 5 beschrieben, geerntet. Mit Hilfe eines Partikelcounters (Beckmann Coulter, Z2) wurde die Zelldichte der Suspension auf 2 x $10^5$ Zellen/ml eingestellt. Anschließend wurde 1 ml dieser Zellsuspension vorsichtig in einem 15 ml Zentrifugationsgefäß auf 5 ml Percoll mit einer Dichte von 1,060 g/ml gegeben und eine Zentrifugation bei 1000 x g und 4°C für 30 Minuten durchgeführt. Danach wurden mit einer 5 ml Pipette zwei 3 ml Fraktionen abgenommen und in jeweils zwei getrennte 15 ml Zentrifugationsgefäße überführt. Die erste Fraktion enthielt die Zellen der Interphase, die zweite Fraktion die Restzellen. Die beiden Fraktionen wurden bei 1000 x g für 10 Minuten zentrifugiert. Anschließend wurde der Überstand dekantiert, die Zellpellets jeweils in 1 ml DPBE resuspendiert und die Zellzahl der beiden Fraktionen in einem Partikelcounter (Beckmann Coulter, Z2) bestimmt. Figur 11 zeigt, daß die untersuchten Zell-Linien von unterschiedlichen Organen, wie Lunge (A549, SCLC-H21), Prostata (PPC-1) Brust (T47D, MDMB 435s) Colon (colo678) und Pankreas (CAPAN-2), eine Dichte von < 1,060 g/ml aufweisen und zu über 90% in der Interphase gefunden werden. Dieser Versuch zeigt, daß zumindest alle Karzinom-Zell-Linien unabhängig ihres Ursprungs eine Dichte aufweisen, die es erlaubt, daß eine Anreicherung dieser Zellen mit dem erfindungsgemäßen Verfahren durchgeführt werden kann.

Beispiel 7

Mit dem erfindungsgemäßen Verfahren wurden bei einer Reihe von Patienten mit verschiedenen manifesten Karzinomen gleichzeitig sowohl immunzytologische/immunzytochemische Untersuchungen als auch eine RT-PCR zum Telomerase-Nachweis durchgeführt. Nach einer Aufklärung und Zustimmung der Patienten wurden bis zu 30 ml Vollblut von der Armvene abgenommen und zwischen 10 bis 20 ml mit dem erfindungsgemäßen Verfahren, wie in Beispiel 5 beschrieben, aufgereinigt und das Zellpellet zweimal in 10 ml PBS gewaschen und zuletzt in 1 ml PBS resuspendiert und 2 Aliquote gebildet. Das erste Aliquot wurde in RNA-Lysispuffer überführt und bis zur RNA-Extraktion und nachfolgender RT-PCR bei -70°C gelagert und die Reaktionen, wie in Beispiel 4 beschrieben, durchgeführt. Das zweite Aliquot wurde den immunzytologischen Färbungen zugeführt. Die Zellzahl die auf einen Objektträger aufgebracht wurden, entsprach dem Äquivalent von 10% der ursprünglich aufgereinigten Blutmenge, d.h. bei 20 ml aufgereinigtem Vollblut wurde das Äquivalent von 2 ml Vollblut konzentriert und auf den Objektträger aufgebracht.

Es wurden routinemäßig gleichzeitig 3 Färbungen durchgeführt: 1. eine Kernfärbung mit einem interkalierenden Farbstoff DAPI, 2. eine Färbung der Epithelzellen mit dem Antikörpercocktail gegen Cytokeratin (Anti-Cytokeratin Cam 5.2, B. D.) und 3. eine Färbung von weißen Blutzellen mit einem Anti-CD45-Antikörper um unspezifische Färbungen auszuschließen.

Die Zellsuspension wurde auf einen mit Poly-L-lysin versetzten Objektträger pipettiert und über Nacht bei 4°C getrocknet. Zur Fixierung der Zellen wurden diese mit 100-200 $\mu$l einer 2%igen Formaldehyd/DPBS-Lösung bei Raumtemperatur inkubiert und anschließend 3x mit DPBS (mit 0,01% NaAz, ohne EDTA) gewaschen. Zur Permeabilisierung der Zellen wurde auf den Objektträger bei Raumtemperatur für 15 Minuten eine 0,5% Triton X-100/DPBS- (mit 0,01% NaAz, ohne EDTA) Lösung gegeben und anschließend wiederum 3 x mit DPBS (mit 0,01% NaAz, ohne EDTA) gewaschen. Zur Blockierung unspezifischer Bindungen und zur Färbung der Zellkerne wurden die Zellen bei Raumtemperatur für 30 Minuten in 2% BSA/DPBS (mit 0,01% NaAz, ohne EDTA) + 1 $\mu$g/ml DAPI inkubiert und 3x mit DPBS (mit 0,01% NaAz, ohne EDTA) gewaschen. 80 $\mu$l des monoklonalen Maus-Anti-Cytokeratin-Antikörpers wurde in einer 1:500-fachen Verdünnung in DPBS (mit 0,01% NaAz, ohne EDTA) für 45 min bei Raumtemperatur auf die Zellen gegeben. Nach dreimaligen Waschen mit DPBS (mit 0,01% NaAz, ohne EDTA) wurde der Objektträger mit 50 $\mu$l eines Phycoerythrin-konjugierten Anti-CD45-Antikörpers (Ziege Anti-Maus-Antikörper) für 45 min bei Raumtemperatur inkubiert und anschließend 3x mit DPBS (mit 0,01% NaAz, ohne EDTA) gewaschen. Nach Hämatoxylinfärbung (50 $\mu$l, 1 min Inkubation) wurden die Objektträger 3x mit $H_2O$ gewaschen und eingedeckt. Zur Kontrolle von unspezifischen Reaktionen wurden stets Präparate gesunder Blutspender mitgeführt.

Die Auswertung dieser Untersuchungen zeigt, daß bei Patienten mit fortgeschrittenen Tumoren des Gastrointestinalbereichs, wie z.B. der Speiseröhre, Magen, Dickdarm, Rectum und Pankreas, sowie in Patienten mit Lungen- und Brusttumoren in 11 von 14 Fällen Cytokeratin-positive CD45-negative epitheliale Zellen im Blut gefunden wurden. Diese Zellen waren Clusterförmig angeordnet und z.T. von CD45-positiven Zellen umgeben, wie es für Zytospinpräparaten von zirkulierenden Tumorzellen typisch ist. Bei diesen Zellen handelt es sich mit hoher Wahrscheinlichkeit um Tumorzellen, da Epithelzellen in dieser Häufigkeit im Blut nicht zu erwarten sind. Die RT-PCR-Untersuchungen waren bei 93% (13/14) dieser Patienten Telomerase-positiv. In Patienten mit lokal begrenzter Erkrankung ohne Anzeichen von Metastasen wurden in 50% der Fälle (3/6) zirkulierende Epithelzellen im Blut gefunden. In 67% dieser Patienten (4/6) konnte die Telomerase nachgewiesen werden. Untersuchungen an gesunden Blutspendern waren Epithelzell- und Telomerase-negativ.

Damit konnte gezeigt werden, daß nicht nur gespikte Tumorzellen verschiedener Zellinien, sondern auch zirkulierende Tumorzellen von Patienten mit unterschiedlichen epithelialen Tumoren (Karzinome) aus dem Blut effizient angereichert werden können.

## Patentansprüche

1. Verfahren zur An- oder Abreicherung von Tumorzellen aus einer Körperflüssigkeit, worin
   ein Zellseparationsmedium mit der Körperflüssigkeit überschichtet und zentrifugiert wird, das Zellseparationsmedium eine Dichte im Bereich von 1,059 bis 1,065 g/ml aufweist,
   die Zentrifugation in einem Gefäß durchgeführt wird, das durch eine poröse Barriere mit einer Porengröße von 20 - 100 $\mu$m in ein oberes und ein unteres Kompartiment geteilt ist, und
   das Zellseparationsmedium im unteren Kompartiment vorgelegt und die Körperflüssigkeit in das obere Kompartiment eingebracht wird.

2. Verfahren nach Anspruch 1, **dadurch gekennzeichnet, dass** das Zellseparationsmedium eine Dichte von 1,059 bis 1,062, insbesondere 1,060 g/ml aufweist.

3. Verfahren nach Anspruch 1, **dadurch gekennzeichnet, daß** die Zentrifugation bei ca. 500 bis 2.000 x g über ca. 10 bis 30 Minuten und bevorzugt bei ca. 1.000 x g über ca. 20 bis 30 Minuten durchgeführt wird.

4. Verfahren nach einem der vorhergehenden Ansprüche, **dadurch gekennzeichnet, daß** das Zellseparationsmedium Percoll oder Ficoll bzw. percoll- oder ficollähnlich ist.

5. Verfahren nach einem der vorhergehenden Ansprüche, **dadurch gekennzeichnet, daß** der Körperflüssigkeit vor dem Überschichten eine oder mehrere Substanzen zugegeben werden, die eine Aggregation von Thrombozyten an Tumorzellen verhindern, und/oder die Körperflüssigkeit vor dem Überschichten von Substanzen befreit wird, die eine Aggregation von Thrombozyten an Tumorzellen fördern.

6. Verfahren nach einem der vorhergehenden Ansprüche, **dadurch gekennzeichnet, daß** die Körperflüssigkeit peripheres Blut ist.

7. Verfahren nach Anspruch 6, **dadurch gekennzeichnet, daß** das periphere Blut in einer gerinnungshemmenden Substanz abgenommen und vor dem Überschichten des Zellseparationsmediums mit einem Verdünnungsmedium verdünnt wird.

8. Verfahren nach Anspruch 7, **dadurch gekennzeichnet, daß** das periphere Blut venöses oder arterielles Blut ist.

9. Verfahren nach einem der Ansprüche 1-5, **dadurch gekennzeichnet, daß** die Körperflüssigkeit ausgewählt ist aus Lymphe, Urin, Exsudaten, Transudaten, Spinalflüssigkeit, Samenflüssigkeit, Speichel, Flüssigkeiten aus natürlichen oder unnatürlichen Körperhöhlen, Knochenmark und dispergiertem Körpergewebe.

**10.** Verfahren nach einem der vorhergehenden Ansprüche, **dadurch gekennzeichnet, daß** das untere Viertel des Zentrifugationsgefäßes nach der Zentrifugation und vor der Abnahme der an Tumorzellen angereicherten Interphase stark abgekühlt wird, um ein Vermischen der Zellen in den verschiedenen Schichten zu verhindern.

**11.** Verfahren nach Anspruch 1, **dadurch gekennzeichnet, daß** die poröse Barriere eine Dicke von 0,5-10 mm, vorzugsweise von 1-5 mm aufweisen.

**12.** Verfahren nach einem der vorhergehenden Ansprüche, **dadurch gekennzeichnet, daß** die poröse Barriere aus einem hydrophoben Material besteht oder mit einem hydrophoben Material beschichtet ist.

**13.** Verfahren nach einem der vorhergehenden Ansprüche, **dadurch gekennzeichnet, daß** das Zellseparationsmedium einen Farbstoff enthält, der das Zellseparationsmedium von der darüberliegenden Körperflüssigkeit farblich unterscheidbar macht und dadurch die Lokalisation der Interphase vereinfacht.

**14.** Verfahren nach einem der vorhergehenden Ansprüche, **dadurch gekennzeichnet, daß** nicht-Tumorzellen, die eine Telomerase-Aktivität aufweisen, von Telomerase-positiven Tumorzellen abgetrennt werden.

**15.** Verfahren zum Nachweis von Tumorzellen in einer Körperflüssigkeit, worin die Tumorzellen durch ein Verfahren gemäß einem der Ansprüche 1-14 angereichert werden.

**16.** Verfahren zur Kultur von Tumorzellen, worin die Tumorzellen durch ein Verfahren gemäß einem der Ansprüche 1-14 angereichert werden.

**17.** Verfahren zur a) An- oder Abreicherung von Tumorzellen aus Blutstammzellen von Knochenmark oder peripherem Blut, oder b) Anreicherung von Blutstammzellen aus Knochenmark oder peripherem Blut, worin in einem ersten Schritt die Blutstammzellen und die Tumorzellen durch ein Verfahren gemäß einem der Ansprüche 1-14 angereichert werden und in einem zweiten Schritt die Blutstammzellen oder die Tumorzellen durch Immunadsorption entweder an- oder abgereichert werden.

**18.** Zentrifugationsgefäß zur An- oder Abreicherung von Tumorzellen aus einer Körperflüssigkeit mit einem Zellseparationsmedium mit einer Dichte von 1,059 - 1,065 g/ml und einer porösen Barriere mit einer Porengröße von 20 - 100 $\mu$m, die das Zentrifugationsgefäß in ein oberes und ein unteres Kompartiment teilt, wobei das Zellseparationsmedium im unteren Kompartiment vorgelegt und die Position der porösen Barriere in dem Zentrifugationsgefäß so gewählt ist, dass der Flüssigkeitsspiegel des Zellseparationsmediums entweder genau unterhalb, genau innerhalb oder genau oberhalb der porösen Barriere zu liegen kommt.

**19.** Kit zur Anreicherung von Tumorzellen aus einer Körperflüssigkeit, umfassend ein Zellseparationsmedium, das eine Dichte im Bereich von 1,059 bis 1,062 g/ml, vorzugsweise von etwa 1,060 g/ml aufweist, sowie ein Zentrifugationsgefäß gemäß Anspruch 18.

**20.** Kit nach Anspruch 19, worin sich das Zellseparationsmedium im unteren Kompartiment des Zentrifugationsgefäßes befindet.

**Claims**

**1.** A method for the enrichment or depletion of tumor cells from a body fluid, in which a cell separation medium is overlaid with the body fluid and centrifuged,
**characterized in that** the cell separation medium has a density in the range from 1.059 to 1.065 g/ml,
**characterized in that** the centrifugation is carried out in a vessel which is divided by a porous barrier having a pore size of 20-100 $\mu$m into an upper and a lower compartment,
the cell separation medium being introduced into the lower compartment, and the body fluid being put in the upper compartment.

**2.** The method as claimed in claim 1, **characterized in that** the cell separation medium has a density in the range from 1.059 to 1.062 g/ml, preferably of about 1.060 g/ml.

3. The method as claimed in claim 1, **characterized in that** the centrifugation is carried out at about 500 to 2000 x g for about 10 to 30 minutes and preferably at about 1000 x g for about 20 to 30 minutes.

4. The method as claimed in any of the preceding claims, **characterized in that** the cell separation medium is Percoll or Ficoll or percoll- or Ficoll-like.

5. The method as claimed in any of the preceding claims, **characterized in that** one or more substances which prevent aggregation of platelets onto tumor cells are added to the body fluid before the overlaying, and/or substances which promote aggregation of platelets onto tumor cells are removed from the body fluid before the overlaying.

6. The method as claimed in any of the preceding claims, **characterized in that** the body fluid is peripheral blood.

7. The method as claimed in claim 6, **characterized in that** the peripheral blood is removed in an anticoagulant substance and is diluted with a diluting medium before overlaying the cell separation medium.

8. The method as claimed in claim 7, **characterized in that** the peripheral blood is venous or arterial blood.

9. The method as claimed in any of claims 1-5, **characterized in that** the body fluid is selected from lymph, urine, exudates, transudates, spinal fluid, seminal fluid, saliva, fluids from natural or unnatural body cavities, bone marrow and dispersed body tissue.

10. The method as claimed in any of the preceding claims, **characterized in that** the lower quarter of the centrifugation vessel is cooled intensively after the centrifugation and before the removal of the interphase enriched in tumor cells, in order to prevent mixing of the cells in the various layers.

11. The method as claimed in claim 1, **characterized in that** the porous barrier has a thickness of 0.5-10 mm, preferably of 1-5 mm.

12. The method as claimed in any of the preceding claims, **characterized in that** the porous barrier consists of a hydrophobic material or is coated with a hydrophobic material.

13. The method as claimed in any of the preceding claims, **characterized in that** the cell separation medium contains a dye which makes it possible to distinguish the cell separation medium from the overlying body fluid by color, and thus simplifies the localization of the interphase.

14. The method as claimed in any of the preceding claims, **characterized in that** non-tumor cells which have a telomerase activity are separated from telomerase-positive tumor cells.

15. A method for detecting tumor cells in a body fluid, in which the tumor cells are enriched by a method as claimed in any of claims 1-14.

16. A method for culturing tumor cells, in which the tumor cells are enriched by a method as claimed in any of claims 1-14.

17. A method for a) the enrichment or depletion of tumor cells from blood stem cells from bone marrow or peripheral blood, or b) the enrichment of blood stem cells from bone marrow or peripheral blood, in which in a first step the blood stem cells and tumor cells are enriched by a method as claimed in any of claims 1-14, and in a second step the blood stem cells or the tumor cells are either enriched or depleted by immunoadsorption.

18. A centrifugation vessel for the enrichment or depletion of tumor cells from a body fluid with a cell separation medium having a density of 1.059-1.065 g/ml and a porous barrier having a pore size or 20-100 $\mu$m, which divides the centrifugation vessel into an upper and a lower compartment, wherein the cell separation medium is introduced into the lower compartment and the position of the porous barrier is chosen such that the liquid level of the cell separation medium is located either exactly below, exactly on the same level as or exactly above the porous barrier.

19. A kit for the enrichment of tumor cells from a body fluid, comprising a cell separation medium which has a density in the range from 1.059 to 1.062 g/ml, preferably of about 1.060 g/ml and a centrifugation vessel according to claim 18.

20. The kit as claimed in claim 19, in which the cell separation medium is present in the lower compartment of the centrifugation vessel.

## Revendications

1. Procédé pour enrichir ou appauvrir des cellules tumorales provenant d'un liquide organique, dans lequel un milieu de séparation cellulaire est recouvert par le liquide organique et centrifugé,
le milieu de séparation cellulaire présente une densité de l'ordre de 1,059 à 1,065 g/ml,
la centrifugation est réalisée dans un récipient, qui est divisé en un compartiment supérieur et un compartiment inférieur par une barrière poreuse présentant une dimension des pores allant de 20 à 100 $\mu$m, et
le milieu de séparation cellulaire est mis dans le compartiment inférieur et le liquide organique est introduit dans le compartiment supérieur.

2. Procédé selon la revendication 1, **caractérisé en ce que** le milieu de séparation cellulaire présente une densité de 1,059 à 1,062, en particulier de 1,060 g/ml.

3. Procédé selon la revendication 1, **caractérisé en ce que** la centrifugation à environ 500 à 2000 g est réalisée pendant environ 10 à 30 minutes et de préférence à environ 1000 g pendant environ 20 à 30 minutes.

4. Procédé selon l'une des revendications précédentes, **caractérisé en ce que** le milieu de séparation cellulaire est le Percoll ou le Ficoll ou similaire à du Percoll ou du Ficoll.

5. Procédé selon l'une des revendications précédentes, **caractérisé en ce qu'**une ou plusieurs substances, qui empêchent une agrégation de thrombocytes aux cellules tumorales, sont ajoutées au liquide organique avant la superposition des couches, et/ou que le liquide organique est libéré des substances, qui favorisent une agrégation de thrombocytes aux cellules tumorales, avant la superposition des couches.

6. Procédé selon l'une des revendications précédentes, **caractérisé en ce que** le liquide organique est du sang périphérique.

7. Procédé selon la revendication 6, **caractérisé en ce que** le sang périphérique est prélevé sur un anticoagulant et dilué à l'aide d'un diluant avant que les couches du milieu de séparation cellulaire soient superposées.

8. Procédé selon la revendication 7, **caractérisé en ce que** le sang périphérique est du sang veineux ou artériel.

9. Procédé selon l'une des revendications 1 à 5, **caractérisé en ce que** le liquide organique est choisi parmi la lymphe, l'urine, les exsudats, les transsudats, le liquide céphalo-rachidien, le liquide séminal, la salive, des liquides issus de cavités corporelles naturelles ou non, la moelle osseuse et le tissu organique dispersé.

10. Procédé selon l'une des revendications précédentes, **caractérisé en ce que** le quart inférieur du récipient de centrifugation est fortement refroidi après la centrifugation et avant la décroissance de l'interphase enrichie en cellules tumorales, pour empêcher qu'un mélange des cellules ne se fasse dans les différentes couches.

11. Procédé selon la revendication 1, **caractérisé en ce que** la barrière poreuse présente une épaisseur de 0,5 à 10 mm, de préférence de 1 à 5 mm.

12. Procédé selon l'une des revendications précédentes, **caractérisé en ce que** la barrière poreuse se compose d'un matériau hydrophobe ou est recouverte d'un matériau hydrophobe.

13. Procédé selon l'une des revendications précédentes, **caractérisé en ce que** le milieu de séparation cellulaire contient un colorant, qui permet de distinguer par la couleur le milieu de séparation cellulaire du liquide organique sus-jacent et qui facilite ainsi la localisation de l'interphase.

14. Procédé selon l'une des revendications précédentes, **caractérisé en ce que** les cellules non tumorales, qui présentent une activité télomérase, sont séparées des cellules tumorales positives à la télomérase.

15. Procédé de dépistage des cellules tumorales dans un liquide organique, dans lequel les cellules tumorales sont enrichies par un procédé selon l'une des revendications 1 à 14.

**16.** Procédé de culture des cellules tumorales, dans lequel les cellules tumorales sont enrichies par un procédé selon l'une des revendications 1 à 14.

**17.** Procédé pour a) enrichir ou appauvrir des cellules tumorales issues de cellules souches du sang de la moelle osseuse ou du sang périphérique, ou b) enrichir des cellules souches du sang issues de la moelle osseuse ou du sang périphérique, dans lequel lors d'une première étape les cellules souches du sang et les cellules tumorales sont enrichies par un procédé selon l'une des revendications 1 à 14 et lors d'une seconde étape les cellules souches du sang ou les cellules tumorales sont soit enrichies, soit appauvries par immunoadsorption.

**18.** Récipient de centrifugation pour enrichir ou appauvrir des cellules tumorales provenant d'un liquide organique à l'aide d'un milieu de séparation cellulaire présentant une densité de 1,059 à 1,065 g/ml et une barrière poreuse présentant une dimension des pores allant de 20 à 100 $\mu$m, qui divise le récipient de centrifugation en un compartiment supérieur et un compartiment inférieur, où le milieu de séparation cellulaire est mis dans le compartiment inférieur et la position de la barrière poreuse est sélectionnée dans le récipient de centrifugation de sorte que le niveau de liquide du milieu de séparation cellulaire se situe soit précisément au-dessous de, précisément dans, soit au-dessus de la barrière poreuse.

**19.** Kit pour enrichir des cellules tumorales provenant d'un liquide organique, comprenant un milieu de séparation cellulaire qui présente une densité de l'ordre de 1,059 à 1,062 g/ml, de préférence d'environ 1,060 g/ml, ainsi qu'un récipient de centrifugation selon la revendication 18.

**20.** Kit selon la revendication 19, dans lequel le milieu de séparation cellulaire se trouve dans le compartiment inférieur du récipient de centrifugation.

Figur 1

Temperaturabhängigkeit der Percolldichte

Dichte/g x ml

Temperatur/°C

Figur 2

Figur 3

Figur 4

A)

gespikte T289$^{GFP}$-Zellen/ml blood
Wiederfindung (Durchfluß Zytometer)
Wiederfindung (Mikroskop)

Zellen

64
32
16
13.75
12.5
10
1.25
8
6.25
2.5
10
12.5
4
2.5

19%    39%    35%    55%    62.5%

Wiederfindung (Durchschnitt)

Figur 5

Vollblut/Ficoll-isolierte PBMC

RNA-Isolierung mit "Vollblut-
Protokoll" oder Standardmethoden
wie Phenol/Chloroform oder
Silicagelsäule

total RNA

Menge RNA die einem
bestimmten Vol. (z.B. 1 ml)
Vollblut entspricht

Buffer: 100 mM Tris/Cl,    DNase-Verdau
              pH 8.3
        50 mM KCl                    30'/37°C, 10'/75°, 10"/90°C
        5 mM MgCl$_2$
        1 mM dNTP-Mix                    → sofort auf Eis
        2.5 µM Random Hexamer
        4U Rnase-freie DNase
        40U RNase Inhibitor
        in 36 µl Volumen

Probe (18 µl)        Negativ-Kontrolle (18 µl)

+ 50U MuLV RT                                    + 4 µl DEPC Wasser
+ 40U Rnase Inhibitor

Reverse Traskriptase Reaktion

                                        30'/42°C, 5'/99°

cDNA              (cDNA)

Buffer: 100 mM Tris/Cl,    PCR-Reaktion [1)]
              pH 8.3
        50 mM KCl
        2 mM MgCl$_2$
        200 µM dNTP-Mix
        2.5U AmpliTaq DNA
              Polymerase   Analyse/Quantifizierung
        300 µM je Primer
        in 25 µl Volumen


1) PCR-Konditionen:

        15"/97°C(15"/97°C,30"/70°C,-0.5°C/cycle,30"/72°C)x10
              (15"/94°C,30"/65°C,-0.5°C/cycle,30"/72°C)x20
              (15"/94°C,30"/50°C,30"(ext.15"/cycle)/72°C)x10
                                          7'/72°C

Figur 6

Wiederfindungsrate gespikter Tumorzellen in Blut

y = 0.7276x
$R^2 = 0.9968$

Anzahl gespikter Zellen

Anzahl wiedergefundener Zellen

Figur 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5663051 A
- US 5840502 A
- US 3741400 A
- US 5487973 A

**In der Beschreibung aufgeführte Nicht-Patentliteratur**

- **KIM et al.** *Science,* 1994, vol. 266, 2011
- **J.A. FLEMING et al.** *J. clin. Path.,* 1967, vol. 20, 145
- Guidelines for the collection, processing and storage of human bone marrow and peripheral stem cells for transplantation, prepared by the BCSH Blood Transfusion Task. *Transfus. Med.,* 1994, vol. 4, 165-72
- **NILSEN, T.W. ; GRAYZEL, J. ; PRENSKY, W.** *J. theor. Biol.,* 1997, vol. 187, 273-284
- **ZHONG et al.** *Tumordiagn. u. Ther.,* 1999, vol. 20, 39
- **BORGEN et al.** International Society for Hematotherapy and Graft Engeneering) Working Group for Standardization of Tumor Cell Detection bei. *Cytotherapy,* 1999, vol. 1, 377
- **CHOMCZYNSKI et al.** *Anal Biochem,* 1987, vol. 162, 156
- **SAMBROOK, J. et al.** Cold Spring Harbor. Cold Spring Harbor Laboratory Press, 1989
- **NAKAMURA et al.** *Science,* 1997, vol. 277, 955-9
- **ALTSCHUL, S. F. et al.** *J Mol Biol,* 1990, vol. 215, 403-410